# EUROPEAN PATENT APPLICATION

(11) **EP 4 610 774 A1**
(43) Date of publication of application: **03.09.2025**
(21) Application number: 25160945.9
(22) Date of filing: 28.02.2025
(51) Int. Cl.: G06F 1/16, A61B 5/00, G04G 21/02, G06F 3/0482, G06F 3/04847, G06F 3/0485

(54) **WEARABLE DEVICE WITH ELECTRONIC USER INPUT UNIT**

(30) Priority: 01.03.2024 US 202463560547 P; 29.03.2024 US 202463571844 P; 25.04.2024 US 202463638799 P; 21.05.2024 US 202463650339 P
(71) Applicant: Masimo Corporation, Irvine, CA 92618 (US)
(72) Inventor: INDORF, Keith Ward, Irvine (US); AL-ALI, Ammar, Irvine (US); AHMED, Omar, Irvine (US); SCRUGGS, Stephen, Irvine (US)
(74) Representative: Finlayson, Scott Henry

(57) **Abstract**

An electronic device can comprise a user input unit protruding from a housing of the electronic device and that is responsive to touch, a display screen, and a processor. The processor can cause the display screen to move display layout previews along a path within the display screen responsive to user input via the user input unit; and responsive to user selection of a display layout preview via the electronic device, render a display layout corresponding to the selected display layout preview, wherein the display layout is larger than the selected display layout preview.

## Description

### TECHNICAL FIELD

The present disclosure relates to electronic devices including wearable devices. Specifically, the present disclosure relates to user input on electronic devices, including a privacy switch for protecting data.

### BACKGROUND

Electronic devices can collect and maintain data which can include private or personal data. Data on an electronic device can be accessible by third-party applications executing on the electronic device or may be accessible to remote computing devices such as if the data is communicated from the electronic device to a remote computing device. Access to data can pose risks to privacy.

### SUMMARY

Disclosed herein is a wearable electronic device for protecting private data, comprising: one or more physiological sensors configured to generate sensor data indicating a physiological status of a user of the wearable electronic device; a device processor electrically connected with the one or more physiological sensors and configured to access the sensor data; an actuator disposed on the wearable electronic device configured to change states responsive to user input; and a controller electrically connected with the device processor and the actuator. The controller can be configured to: monitor the state of the actuator; responsive to determining that the actuator is in a privacy state, communicate a notification signal to the device processor to cause the device processor to prepare for a privacy mode, wherein the device processor is configured to terminate one or more processes responsive to the notification signal from the controller to prepare for the privacy mode; delay implementing the privacy mode until one or more conditions have been satisfied to allow the device processor to prepare for the privacy mode pursuant to communicating the notification signal to the device processor; and implement the privacy mode to inhibit access to the sensor data responsive to satisfaction of the one or more conditions.

In some implementations, the device processor is configured to provide confirmation to the controller when the one or more processes have been terminated, wherein the one or more conditions comprise receiving the confirmation at the controller from the device processor that the one or more processes have been terminated.

In some implementations, the controller is configured to initiate a timer pursuant to determining that the actuator is in the privacy state, wherein the one or more conditions include the timer exceeding a threshold.

In some implementations, the controller is configured to initiate the timer in response to communicating the notification signal to the device processor.

In some implementations, the controller is configured to implement the privacy mode responsive to the timer exceeding the threshold regardless of whether the one or more processes have been terminated.

In some implementations, the controller is configured to implement the privacy mode based on disconnecting a privacy component of the wearable electronic device from a power source, the privacy component comprising one or more of a communication component, a GNSS component, a microphone, a camera, or an altimeter.

In some implementations, the controller is configured to implement the privacy mode based on holding a privacy component of the wearable electronic device in a reset mode, the privacy component comprising one or more of a communication component, a GNSS component, a microphone, a camera, or an altimeter.

In some implementations, the controller is non-programmable.

In some implementations, the device processor is electrically isolated from the actuator and does not read the state of the actuator.

In some implementations, the wearable electronic device comprises a display screen configured to display indicia responsive to implementing the privacy mode, wherein the display screen is configured to display the indicia throughout the privacy mode.

In some implementations, the controller is configured to cause the indicia to be displayed on the display screen.

Disclosed herein is a method of implementing privacy on an electronic device, comprising: generating sensor data with one or more physiological sensors, the sensor data indicating a physiological status of a user of the electronic device; causing an actuator of the electronic device to change states responsive to user input; monitoring the state of the actuator with a controller electrically connected with the actuator; responsive to determining that the actuator is in a privacy state, communicating a notification signal from the controller to a device processor to cause the device processor to prepare for a privacy mode; terminating one or more processes with the device processor responsive to the notification signal from the controller to prepare for the privacy mode; delaying implementing the privacy mode until one or more conditions have been satisfied to allow the device processor to prepare for the privacy mode; and implementing the privacy mode to inhibit access to the sensor data responsive to satisfaction of the one or more conditions.

In some implementations, the one or more conditions comprise receiving confirmation at the controller from the device processor that the one or more processes have been terminated.

In some implementations, the method further comprises initiating a timer pursuant to determining that the actuator is in the privacy state, wherein the one or more conditions include the timer exceeding a threshold.

In some implementations, the method further comprises implementing the privacy mode based on disconnecting a privacy component of the electronic device from a power source, the privacy component comprising one or more of a communication component, a GNSS component, a microphone, a camera, or an altimeter.

In some implementations, the method further comprises implementing the privacy mode based on holding a privacy component of the electronic device in a reset mode, the privacy component comprising one or more of a communication component, a GNSS component, a microphone, a camera, or an altimeter.

Disclosed herein is non-transitory computer-readable media including computer-executable instructions that, when executed by a computing system, cause the computing system to perform operations comprising: monitoring a state of an actuator configured to change states responsive to user input; responsive to determining that the actuator is in a privacy state, causing a device processor to prepare for a privacy mode by terminating one or more processes; delaying implementing the privacy mode until one or more conditions have been satisfied to allow the device processor to terminate the one or more processes; and implementing the privacy mode to inhibit access to sensor data responsive to satisfaction of the one or more conditions.

In some implementations, the computer-executable instructions, when executed by the computing system, cause the computing system to perform operations comprising: implementing the privacy mode when the one or more processes have been terminated, wherein the one or more conditions include termination of the one or more processes.

In some implementations, the computer-executable instructions, when executed by the computing system, cause the computing system to perform operations comprising: initiating a timer pursuant to determining that the actuator is in the privacy state; implementing the privacy mode when the timer exceeds a threshold, wherein the one or more conditions include the timer exceeding the threshold.

In some implementations, the computer-executable instructions, when executed by the computing system, cause the computing system to perform operations comprising implementing the privacy mode based on disconnecting a privacy component from a power source and/or holding the privacy component in a reset mode, the privacy component comprising one or more of a communication component, a GNSS component, a microphone, a camera, or an altimeter.

Disclosed herein is an electronic device comprising: one or more physiological sensors configured to generate sensor data indicating a physiological status of a user of the electronic device; and one or more hardware processors that can be configured to: determine whether a privacy mode is executing on the electronic device based on monitoring a state of an actuator, wherein one or more communication components are disabled during the privacy mode to inhibit communication of the electronic device; responsive to determining that the privacy mode is executing, cause the one or more physiological sensors to generate the sensor data during the privacy mode to measure a physiological status of the user during the privacy mode; obfuscate the sensor data with one or more encryption protocols and an encryption key to inhibit program applications without access to the encryption key from decrypting the sensor data; and modify metadata of the sensor data to indicate a privacy status of the sensor data, the privacy status corresponding to whether the sensor data was generated during the privacy mode.

In some implementations, the one or more hardware processors are configured to cause the one or more communication components to communicate the sensor data from the electronic device to a receiving electronic device during a non-privacy mode.

In some implementations, the one or more hardware processors are configured to cause the one or more communication components to communicate the sensor data over a secure channel.

In some implementations, the one or more hardware processors are configured to cause the one or more communication components to communicate the sensor data with the metadata.

In some implementations, the one or more hardware processors are configured to cause the one or more physiological sensors to generate the sensor data during the privacy mode responsive to determining that the user has selected to generate the sensor data during the privacy mode.

In some implementations, the one or more hardware processors are configured to allow the sensor data to be erased from short-term storage without storing the sensor data as persistent data responsive to determining that the user has not selected to store the sensor data.

In some implementations, the one or more hardware processors are configured to store the sensor data responsive to determining that the user has selected to store the sensor data.

In some implementations, the one or more hardware processors are configured to store the sensor data as persistent data in long-term storage.

In some implementations, the one or more hardware processors are configured to store the sensor data in a first location in storage and store non-private sensor data generated during a non-privacy mode in a second location in storage.

In some implementations, the one or more hardware processors are configured to store the sensor data in a same partition of storage as non-private sensor data generated during a non-privacy mode.

In some implementations, the one or more hardware processors are configured to store the sensor data in a structured data format based on the privacy status of the sensor data.

In some implementations, the one or more hardware processors are configured to: store the sensor data as historical sensor data; access the historical sensor data from storage; determine a privacy status of the historical sensor data from the metadata; and generate user interface data for rendering a display comprising indicia of the historical sensor data and an indication of the privacy status of the historical sensor data.

In some implementations, the one or more hardware processors are configured to obfuscate non-private sensor data generated during a non-privacy mode with another encryption key different than the encryption key.

In some implementations, the one or more hardware processors are configured to obfuscate non-private sensor data generated during a non-privacy mode with another encryption protocol different than the one or more encryption protocols.

Disclosed herein is an electronic device that can comprise: one or more physiological sensors configured to generate sensor data indicating a physiological status of a user of the electronic device; and one or more hardware processors that can be configured to: access the sensor data; determine whether the sensor data was generated during a privacy mode based on metadata of the sensor data, the metadata indicating the sensor data is private if the sensor data was generated during the privacy mode; determine whether the user has selected to enable private data to be communicated from the electronic device; and communicate the sensor data from the electronic device to a receiving computing device over a secure channel responsive to determining that the sensor data is private and that the user has selected to enable private data to be communicated.

In some implementations, the one or more hardware processors are configured to inhibit the sensor data from being communicated from the electronic device to the receiving computing device responsive to determining that the sensor data is private and that the user has not selected to enable private data to be communicated.

In some implementations, the one or more hardware processors are configured to encrypt the sensor data.

In some implementations, the one or more hardware processors are configured to: implement a key exchange protocol to generate a key; and communicate the sensor data over the secure channel with the key.

In some implementations, the one or more hardware processors are configured to communicate the sensor data over the secure channel by communicating the sensor data to a trusted third-party device.

In some implementations, the one or more hardware processors are configured to inhibit program applications executing on the electronic device from accessing the sensor data responsive to determining that the sensor data is private.

Disclosed herein is an electronic device configured to monitor physiological parameters of a user. The electronic device can comprise: a sensor assembly integrated with the electronic device, the sensor assembly comprising one or more physiological sensors configured to generate physiological data of the user; a user input unit protruding from a housing of the electronic device, wherein the user input unit is contiguously integrated with an exterior surface of the housing and forms a portion of the exterior surface of the housing, wherein the user input unit is responsive to touch from the user; a display screen connected to the housing, wherein the user input unit is positioned on the housing out of a plane on which the display screen lies; and a processor in electrical communication with the display screen, the one or more physiological sensors, and the user input unit. The processor can be configured to cause the display screen to: display a plurality of display layout previews, wherein the display screen is configured to display less than all of the display layout previews at a time, wherein the display layout previews provide visualizations of display layouts on a reduced scale of size; move the display layout previews along a path within the display screen responsive to user input via the user input unit, wherein the path comprises an arc of a circle with a center that is offset from a center of the display screen, the center of the display screen being positioned between the center of the circle and the user input unit; and responsive to user selection of a display layout preview via the electronic device, render a display layout corresponding to the selected display layout preview, wherein the display layout is larger than the selected display layout preview, the display layout comprising indicia of the physiological data originating from the one or more physiological sensors arranged within the display layout according to the selected display layout preview.

In some implementations, the user input unit does not move relative to the housing.

In some implementations, the display screen is configured to display no more than two of the display layout previews at a time.

In some implementations, the user input unit is positioned on the housing to the right of the display screen.

In some implementations, the processor is configured to receive the user selection of the display layout preview via the user input unit.

In some implementations, the processor is configured to receive the user selection of the display layout preview via the display screen.

In some implementations, the user input via the user input unit comprises user contact sliding across the user input unit.

In some implementations, the user input unit comprises a plurality of zones independently responsive to touch from the user, wherein the processor is configured to cause the display screen to move the display layout previews along the path based on user interaction with the plurality of zones.

In some implementations, the processor is configured to cause the display screen to move the display layout previews along the path with a speed that depends on time between user interactions with the plurality of zones.

In some implementations, the processor is configured to cause the display screen to move the display layout previews along the path in a direction that depends on an order in which the user interacts with the plurality of zones.

In some implementations, the user input unit has a length between about 25% and about 75% a diameter of the display screen.

In some implementations, the user input unit has a height between about 0.4cm and 0.6cm.

In some implementations, the user input unit is one of a capacitive, resistive, infrared, surface capacitive, or projected capacitive touch (PCAP) user input unit.

Various combinations of the above and below recited features, embodiments, implementations, and aspects are also disclosed and contemplated by the present disclosure.

Additional implementations of the disclosure are described below in reference to the appended claims, which may serve as an additional summary of the disclosure.

In various implementations, systems and/or computer systems are disclosed that comprise a computer-readable storage medium having program instructions embodied therewith, and one or more processors configured to execute the program instructions to cause the systems and/or computer systems to perform operations comprising one or more aspects of the above- and/or below-described implementations (including one or more aspects of the appended claims).

In various implementations, computer-implemented methods are disclosed in which, by one or more processors executing program instructions, one or more aspects of the above- and/or below-described implementations (including one or more aspects of the appended claims) are implemented and/or performed.

In various implementations, computer program products comprising a computer-readable storage medium are disclosed, wherein the computer-readable storage medium has program instructions embodied therewith, the program instructions executable by one or more processors to cause the one or more processors to perform operations comprising one or more aspects of the above- and/or below-described implementations (including one or more aspects of the appended claims).

### BRIEF DESCRIPTION OF THE DRAWINGS

Various implementations will be described hereinafter with reference to the accompanying drawings. These implementations are illustrated and described by example only, and are not intended to limit the scope of the disclosure. In the drawings, similar elements may have similar reference numerals.
FIGS. 1A-1F illustrate an example electronic device with user input including a privacy switch.
FIG. 2 is a schematic block diagram illustrating an example implementation of an electronic device in communication with one or more remote devices.
FIG. 3 illustrates a schematic system diagram of an electronic device including a sensor assembly and user input unit.
FIGS. 4A-4D illustrate example user interfaces of an electronic device.
FIG. 5 is a front view of the example electronic device according to aspects of the disclosure.
FIG. 6A-6B are perspective views of example implementations of electronic devices.
FIG. 7 is a block diagram of an example electronic device.
FIGS. 8A-8C are block diagrams illustrating example implementations of an electronic device.
FIG. 9A is a flowchart illustrating an example process of implementing a privacy mode on an electronic device.
FIG. 9B is a flowchart illustrating an example process of managing device data generating during privacy modes and non-privacy modes.
FIG. 9C is a flowchart illustrating an example process of managing access to and communication of device data.
FIG. 10 is a perspective view illustrating an example implementation of an actuator of an electronic device.
FIGS. 11A-11D illustrate example displays of an electronic device which may display user interfaces.
FIG. 12 illustrates an example electronic device with a privacy switch implemented as a sound bar.
FIG. 13 illustrates an example electronic device with a privacy switch implemented as a phone.
FIG. 14 illustrates an example electronic device with a privacy switch implemented as a monitoring hub.

### DETAILED DESCRIPTION

The present disclosure will now be described with reference to the accompanying figures, wherein like numerals may refer to like elements throughout. The following description is merely illustrative in nature and is in no way intended to limit the disclosure, its application, or uses. Furthermore, the devices, systems, and/or methods disclosed herein can include several novel features, no single one of which is solely responsible for its desirable attributes or which is essential to practicing the devices, systems, and/or methods disclosed herein. Additionally, the structures, systems, and/or devices described herein may be embodied as integrated components or as separate components.

Some aspects and/or implementations have been described in connection with the accompanying drawings. The figures may be drawn to scale, but such scale is not limiting, since dimensions and proportions other than what are shown are contemplated and are within the scope of the disclosed invention. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, any methods described herein may be practiced using any device suitable for performing the recited steps. Various steps within a method may be executed in different order without altering the principles of the present disclosure.

A dedicated privacy button for electronic device or wireless communications device (for example, cell phone, smart watch, etc.) can permit a user to control if and/or when third party apps, such as apps downloaded from an app store) are allowed to access private or sensitive data, such as health data. Some legitimate third party apps are given permission (for example, by the user) to access a user's health data via an API. However, the permission is typically "all or nothing." Disclosed herein is a dedicated privacy button (for example, a hardware switch, a software switch, etc.) that allows a user to activate a privacy window (for example, a particular duration of time) over which little or no health data is shared (for example, with any apps, or with a particular app). Disclosed herein is an electronic device that includes software, firmware, hardware, or any combination thereof to provide or facilitate healthcare related services or applications. The electronic device may include hardware or software configurations that allows a user to adjust privacy-related settings, for example restricting private and personal data.

An electronic device can include any of the example electronic devices shown and/or described herein or can include one or more features or components thereof. For example, the electronic device can be implemented as a wearable device, smart watch, cell phone, smart phone, tablet computer, desktop computer, laptop, mobile computing device, mobile phone, personal digital assistant (PDA), hybrid PDA/mobile phone, any electronic device configured to communicate over a network, or any device configured for the internet of things.

The health care module can be configured to obtain or generate heath data from a user of the wireless communicates device. For example, the health care module can include one or more sensors configured to obtain physiological data. In some cases, the electronic device 10 includes an emitter and a detector for obtaining one or more physiological measurements. In some cases, the health data is private, sensitive, confidential and/or HIPPA protected.

The electronic device can include one or more processors. The one or more processors can be configured to implement a third party application, such as a mobile app. In some cases, the user can adjust privacy-related settings (for example, permissions) for third party applications. For example, in some cases, the user can choose to prevent the third party application from obtaining, or having access to, private or sensitive data, such as health data. Alternatively, in some cases, the user can choose to allow the third party application to obtain, or have access to, private or sensitive data. In some cases, the electronic device includes a dedicated privacy switch that is implemented in hardware and/or software. In some cases, activation of the dedicated privacy switch can cause the electronic device to prevent the third party application from obtaining the private or sensitive data. For example, for data that is generated while the privacy switch is activated, the third party application may not have access to such data while the privacy switch is activated and/or after the privacy switch is deactivated. In some implementations, deactivation of the dedicated privacy switch can cause the electronic device to allow the third party application to obtain or access the private or sensitive data. In some cases, activation of the dedicated privacy switch can cause the electronic device to temporarily prevent the third party application from obtaining the private or sensitive data for a first period of time (for example, 30 minutes, 1 hour, 8 hours, 24 hours, 1 week, etc.). In some cases, the third party application is automatically allowed to resume obtaining the private or sensitive data after the expiration of the first period of time. Furthermore, in some cases, deactivation of the dedicated privacy switch can cause the electronic device to temporarily allow the third party application to obtain the private or sensitive data for a second period of time. In some cases, the third party application is automatically prevented from obtaining the private or sensitive data after the expiration of the second period of time.

The electronic device can identify some or all of the private or sensitive data, including data obtained or generated by the health care module. For example, the electronic device can associate an attribute (for example, use metadata to tag) with all of the private or sensitive data obtained or generated by the health module. In some such cases, in response to the activation of the dedicated privacy switch and for the duration of the first period of time, the electronic device can prevent transmission of or access to any data associated with the attribute. In some cases, the private or sensitive data is restricted by default. A user can give permission sell the private or sensitive data to the third party application. In some cases, the user shares in income from the sale of the private or sensitive data.

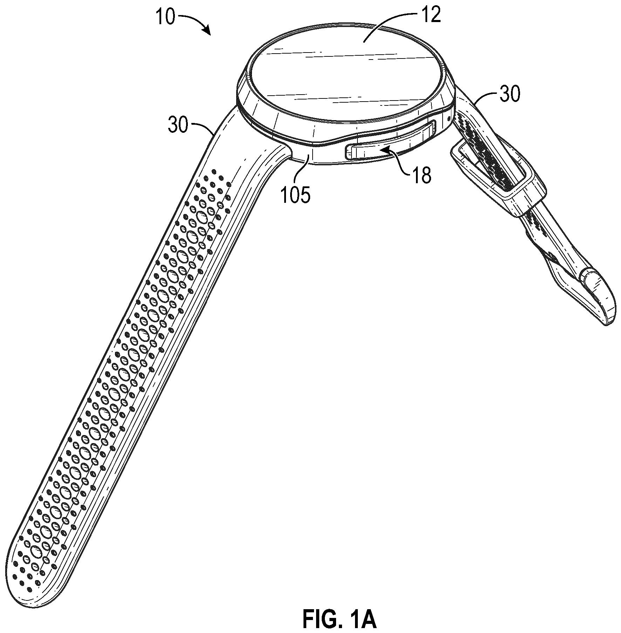

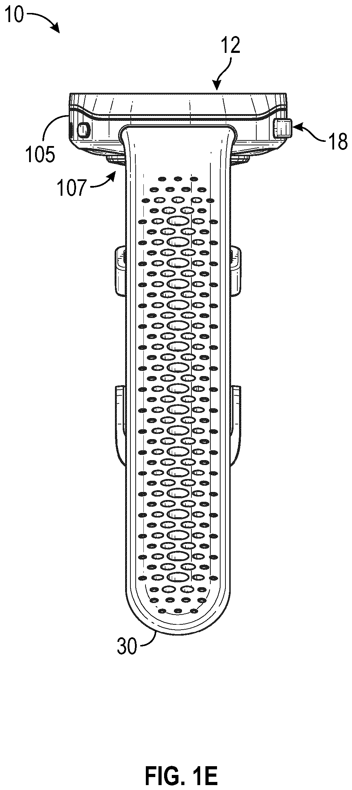

FIGS. 1A-1F illustrate perspective views of an example electronic device 10. The electronic device 10 may be a wearable device such as a smartwatch. FIGS. 1A-1F are not intended to be limiting of the present disclosure. One or more of any of the structural and/or operational features shown and/or discussed with respect to electronic device 10, or any of the other electronic devices herein, can be implemented in any of the example electronic and/or computing devices shown and/or discussed herein such as a phone, a laptop, a computer, a tablet, or any other computing device.

The electronic device 10 can include a display screen 12, housing 105, and one or more straps 30. The straps 30 can be adjustable. The straps 30 can be configured to secure the electronic device 10 to a body part of a user of the electronic device 10, such as a wrist. The display screen 12 can be an LED display. The display screen 12 can be configured to display day, month, date, year, and/or time. The display screen 12 can display time as analog or digital. The display screen 12 can display physiological related data such as physiological parameters, physiological trends, physiological graphs, or the like. For example, the display screen 12 can display heart rate, respiration rate, ECG data, SpO2, a step count of the number of steps taken by a user of the electronic device 10, or the like. The housing 105 can be circular and can surround the display screen 12. The housing 105 can be formed of an electrically conductive material, such as metal. The housing 105 can include one or more electrodes for monitoring cardiac electrical activity.

The electronic device 10 can include a user input unit 18 which may be positioned on, or coupled to, the housing 105 of the electronic device 10. In some examples, the user input unit 18 may be in the shape of a square, rectangular, circle, and/or any other geometric and/or irregular shape. The user input unit 18 may be generally curved and follow a circular curvature of the housing 105 as depicted in FIGS. 1A-1F. In some examples, a user input unit 18 may be fixed to the housing 105 such that user input unit 18 does not rotate, slide, or otherwise move relative to the housing 105. The user input unit 18 protrudes from an exterior surface of the housing, protruding from a cavity along an exterior surface of the housing, and/or the like. The user input unit 18 is contiguously integrated with the housing 105, or exterior surface thereof such that the outer surfaces of the user input unit 18 and housing 105 form a single unitary surface. The user input unit 18 can comprise an electrically conductive material such as metal. The user input unit 18 can comprise a non-conductive material such as glass or plastic and which may be responsive to changes in capacitance. In some implementations, the user input unit 18 can comprise a physical vapor deposition (PVD) coating layer. In some implementations, electronic device 10 can include a plurality of user input units 18 (e.g., two, three, four, five, and/or more).

In some examples, the user input unit 18 may be located on a right side of the housing 105 between straps 30. Typically, a user input unit 18 may be placed on the right side of a housing 105 as viewed from the display screen 12, as users may generally wear a watch on their left wrist. In this configuration, a user may use a finger on their right hand to operate the user input unit 18. Additionally and/or alternatively, the user input unit 18 may be positioned at another location on the housing 105 and/or the electronic device 10. In some examples, one user input unit 18 may be located on the left side of the housing, between straps 30, while one or more user input units 18 are positioned at other locations (e.g., the face, the bottom, a second user input unit 18 on a same side, a second user input unit 18 on an opposite side, and/or the like). Optionally and/or alternatively, the user input unit 18 may include one or more of an actuator, a button, a switch and/or the like, in combination and/or in addition to a user input unit 18.

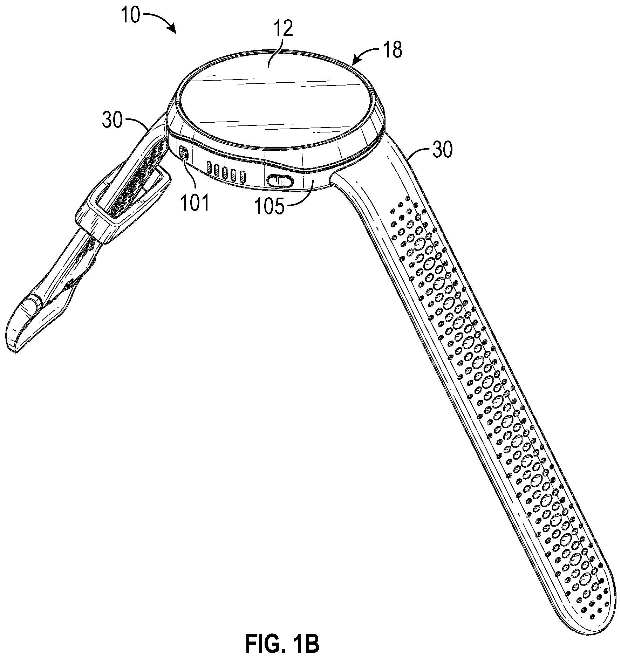

As depicted in FIG. 1B, electronic device 10 can include an actuator 101. The actuator 101 can include one or more physical components. For example, the actuator 101 can be a switch, a lever, a button, a dial, a knob, a rocker, a toggle, or the like. The actuator 101 can be a slideable switch. The actuator 101 can include one or more electrical components. For example, the actuator 101 can be a capacitive touch screen. In some implementations, the actuator 101 can be a button displayed on a touch screen, a slider displayed on a touch screen, or the like.

In some implementations, the actuator 101 can include a camera configured to capture images and a hardware processor configured to perform one or more image processing techniques such as facial recognition. In some implementations, the actuator 101 can include a microphone configured to capture audio input and a hardware processor configured to perform one or more audio processing techniques such as voice recognition.

The actuator 101 can transition between states such as a first state and second state. In some implementations, the states represent a physical state (for example, switch is in one position or another position). In some implementations, the states represent an electrical state (for example, change in electrical capacitance, resistance, or the like). The actuator 101 can be configured to actuate (for example, transition between states) in response to a physical input, such as a motion to slide a switch, depress a button, or the like. The actuator 101 can be configured to actuate (for example, transition between states) in response to an electrical input, such as a change in capacitance, voice recognition, facial recognition, or the like.

The actuator 101 can be located on a housing 105 of the electronic device 10. The actuator 101 can be adjacent to the display screen 12. In some implementations, the actuator 101 can be included on the display screen 12, such as a portion of the display screen 12, for example, in implementations where the actuator 101 is a capacitive touchscreen or portion thereof.

A user can actuate the actuator 101 to cause the electronic device 10 to transition between a privacy mode and non-privacy mode. For example, the actuator 101, when in a first state, can cause the electronic device 10 to enter a privacy mode, and the actuator 101, when in a second state, can cause the electronic device 10 to enter a non-privacy mode. The electronic device 10, or components thereof, can operate differently when in the privacy mode than when in the non-privacy mode. For example, one or more components of the electronic device 10, such as a communication component, may not operate during a privacy mode. As another example, a hardware processor of the electronic device 10 can handle data, such as sensor data, differently during a privacy mode than during a non-privacy mode. The electronic device 10 can operate normally during a non-privacy mode.

In some implementations, the actuator 101 can cause the electronic device 10 to only transition between the privacy mode and non-privacy mode. For example, the actuator 101 can be dedicated to transitioning the electronic device 10 between privacy and non-privacy modes and may not serve any other purpose. Advantageously, an actuator 101 dedicated to only causing the electronic device 10 to transition between privacy and non-privacy states can provide an improved user experience for transitioning the electronic device between privacy and non-privacy modes by providing a simple and easy-to-understand process that does not involve determining which operations actuating the actuator 101 will affect, such as in implementations where the actuator 101 can affect other operations of the electronic device in addition to transitioning between privacy and non-privacy modes. In some implementations, the actuator 101 can cause to electronic device 10 perform other operations in addition to transitioning between privacy and non-privacy modes. For example, in some implementations, actuating the actuator 101 can also cause the electronic device to power on/off, can turn the display screen 12 on/off, or the like.

In some implementations, the actuator 101 can be a single component. In some implementations, the actuator 101 can be the only component necessary to transition the electronic device 10 between privacy and non-privacy modes. For example, a user may not need to perform any other operation, other than actuating the actuator 101, to transition the electronic device 10 between privacy and non-privacy modes. Advantageously, the ability to transition the electronic device 10 between privacy and non-privacy states by only actuating the actuator 101 can provide an improved user experience for transitioning the electronic device between privacy and non-privacy modes by providing a simple and easy-to-understand process that does not involve multiple operations. For example, the privacy switch does not require actuating multiple actuators simultaneously or sequentially. In some implementations, more than one operation may be required to transition the electronic device 10 between privacy and non-privacy modes. This can include actuating more than one actuator.

Advantageoulsy, a user may be able to easily toggle the electronic device 10 between privacy modes and non-privacy modes by actuating the actuator 101.

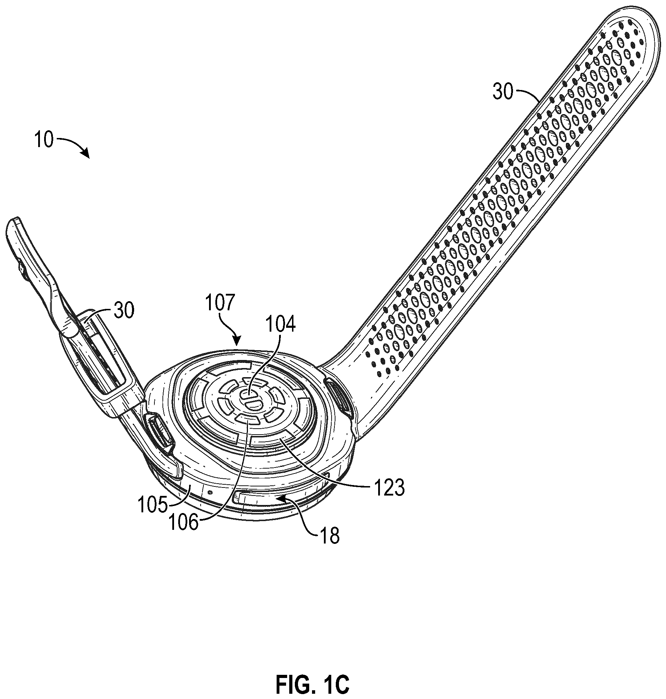

As depicted in FIG. 1C, a bottom side of a wearable electronic device 10 can include a sensor assembly 107 which can include emitters 104, detectors 106, and/or electrodes 123. The electrode(s) 123 can be configured to contact the skin of a user of the electronic device 10. The electrodes 123 can be configured to measure electrical activity. The electrode(s) 123 can obtain data related to the cardiac activity of a user of the electronic device 10, such as ECG data. The emitter(s) 104 can include one or more light emitting diodes (LEDs). The emitter(s) 104 can emit light of various wavelengths which can penetrate into a tissue of the user of the electronic device 10. The detector(s) 206 can detect light emitted by the emitter(s) 104 and attenuated by the tissue of a wearer. The attenuation of light can be via transreflectance by the user's body tissue, for example, by the pulsatile arterial blood flowing through the capillaries (and optionally also the arteries) within a tissue site where the electronic device 10 is worn (for example, the wrist). The detector(s) 106 can generate one or more signals based at least in part on the light detected that was emitted by the emitter(s) 104. The detector(s) 106 can generate data relating to blood oxygen saturation of a user of the electronic device 10. The detector(s) 106 can generate data relating to spectroscopy.

The wearable electronic device 10 can measure an indication of the wearer's physiological parameters, which can include, for example, pulse rate, respiration rate, oxygen saturation (SpO2), Pleth Variability Index (PVI), Perfusion Index (PI), Respiration from the pleth (RRp), hydration, glucose, blood pressure, and/or other parameters. The wearable electronic device 10 can calculate a wellness index based on more than one individual physiological parameter measured by the wearable electronic device 10 and/or received by the wearable electronic device 10. The wearable electronic device 10 can perform intermittent and/or continuous monitoring of measured parameters. The sensors can additionally and/or alternatively perform a spot check of the measured parameters, for example, upon request by the wearer.

A strap 30 can be stretchable and evenly distribute the pressure of the electronic device 10 around the wrist so as to provide better contact between sensor assembly 107 and the wrist while not compromising the comfort of the wearer and/or reducing the blood flow across the wrist in a way that reduces the accuracy of the measurement by sensor assembly 107. In some examples, the strap 30 can include a rubber base. The rubber base can be molded through a plurality of metal loops arranged along a length of strap 30 to form the strap 30.

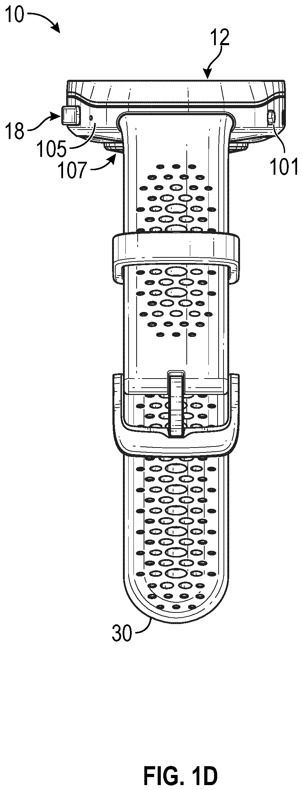

As depicted in FIG. 1D, the actuator 101 can be located on a housing 105 of the electronic device 10. The sensor(s) of the electronic device 10, such as the electrode(s) 123, the emitter(s) 104, and/or the detector(s) 106, can be located on a housing 105 of the electronic device 10. The actuator 101 can be located on a different portion of the electronic device 10 housing105 than one or more of the sensors. The actuator 101 may not be in physical contact with one or more of the sensors of the electronic device 10. In some implementations, the actuator 101 can be located on a portion of the electronic device 10 housing 105 that is accessible to a user to actuate the actuator 101. In some implementations, one or more of the sensors of the electronic device 10 can be located on a portion of the electronic device 10 housing 105 that is not readily accessible to user. For example, a user may not be able to access the one or more sensors with their fingers. For example, the sensors can be located on an underside portion of the electronic device 10 such that when the electronic device 10 is worn by a user, the electronic device 10 covers the sensors against the user, such as against a wrist area of the user such that the user may not be able to readily access the sensors with their fingers. Advantageously, a user can be able to easily actuate the actuator 101 without interfering with or disrupting the sensors, such as data collection of the sensors, because the actuator 101 and sensors are located on different portions of the electronic device 10 housing, for example. For example, the sensors can continue to operate when a user actuates the actuator 101 between states. In some implementations, the actuator 101 may not cover or block one or more of the sensors when the actuator is in any of the actuator states.

In some implementations, the actuator 101 can be located on a same portion of electronic device 10 housing 105 as some sensors and a different portion of the electronic device 10 housing 105 than other sensors. In some implementations, some sensors can be located on a different portion of the electronic device 10 housing 105 than other sensors

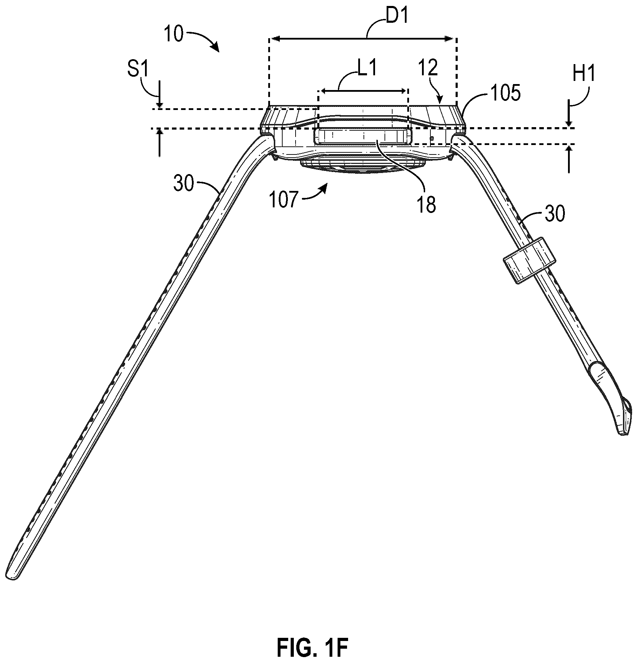

As shown in FIG. 1F, for example, the user input unit 18 can have a length L1 extending along the housing 105 (e.g., between the straps 30). The length L1 of the user input unit 18 can be between 1.5cm and 3.0cm, between 1.75cm and 2.75cm, between 2.0cm and 2.5cm, or about 2.25 cm. The length L1 of the user input unit 18 can be between 25% and 100% the diameter D1 of the display screen 12, between 25% and 75% of D1, between 25% and 50% of D1, between 50% and 100% of D1, between 50% and 75% of D1, or any other ratio therebetween. In some implementations, the length L1 of the user input unit 18 can be greater than of D1 such as if the user input unit 18 wraps around the circumference of the display screen 12. The user input unit 18 can have a height H1 extending from the top from the top of the housing 105 to the bottom of the housing, with respect to the page of FIG. 1F. The height H1 of the user input unit 18 can be between 0.25cm and 1.0cm, between 0.25cm and 0.75cm, between 0.35cm and 0.65cm, between 0.4cm and 0.6cm, or about 0.5cm. The height H1 and/or length L1 of the housing can be uniform such that a profile of the user input unit is rectangular from the view shown in FIG. 1F. FIG. 1F may be drawn to scale.

Distance S1 represents a distance between user input unit 18 and a plane in which display screen 12 lies. In this case, the display screen 12 is represented as being below the top part of housing 105. In some cases, display screen 12 may be flush with the top part of housing 105. Thus, the distance S1 may vary depending on the implementation. As represented by distance S1, user input unit 18 is out of the plane in which the display screen 12 lies such that the plane in which display screen 12 lies does not intersect user input unit 18. User input unit 18 is beneath the display screen 12 such that user input unit 18 is between the plane of the display screen 12 and the user's wrist when the electronic device 10 is worn by the user.

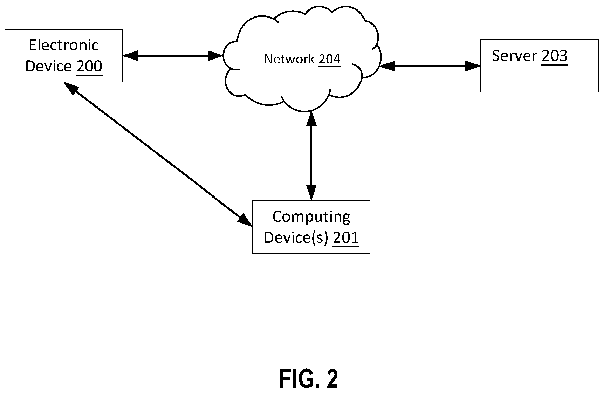

FIG. 2 is a schematic block diagram illustrating an example implementation of an electronic device 200 in communication with one or more devices remote to the electronic device 200 via a network 204. Electronic device 200 can be implemented as any of the other example electronic devices shown and/or described herein. Electronic device 200 can communicate with one or more servers 203 via network 204. The network 204 can include one or more communications networks. The network 204 can include a plurality of computing devices configured to communicate with one another. The network 204 can include the Internet. The network 204 can include any combination of a body area network (e.g., implementing human body communication with capacitive coupling via the tissue of a user's body), a local area network ("LAN") and/or a wide area network ("WAN"), or the like. Accordingly, various computing devices can communicate with one another directly or indirectly via any appropriate communications links and/or networks, such as network 204 (e.g., one or more communications links, one or more computer networks, one or more wired or wireless connections, the Internet, any combination of the foregoing, and/or the like). The electronic device 200 can, via the network 204, communicate data to the server 203 and/or receive data from the server 203, such as sensor data which can include physiological data.

In some implementations, one or more aspects of electronic device 200 can be open (e.g., open source and/or including open architecture) to allow third party access to aspects of the electronic device 200 such as physiological data or other sensor data. Third party applications can be installed on the electronic device 200 such as from the server 203. In some cases, third party applications can access data on the electronic device 200 such as physiological data from sensors on the electronic device 200.

The server 203 can comprise one or more computing devices including one or more hardware processors. The server 203 can comprise program instructions configured to cause the server 203 to perform one or more operations when executed by the hardware processors. The server 203 can include, and/or have access to (e.g., be in communication with and/or host) a storage device, database, or system which can include any computer readable storage medium and/or device (or collection of data storage mediums and/or devices), including, but not limited to, one or more memory devices that store data, including without limitation, dynamic and/or static random-access memory (RAM), programmable read-only memory (PROM), erasable programmable read-only memory (EPROM), electrically erasable programmable read-only memory (EEPROM), optical disks (e.g., CD-ROM, DVD-ROM, etc.), magnetic disks (e.g., hard disks, floppy disks, etc.), memory circuits (e.g., solid state drives, random-access memory (RAM), etc.), and/or the like. In some implementations, the server 203 can include and/or be in communication with a hosted storage environment that includes a collection of physical data storage devices that may be remotely accessible and may be rapidly provisioned as needed (commonly referred to as "cloud" storage). Data stored in and/or accessible by the server 203 can include sensor data, such as physiological data, including historical physiological data previously obtained by one or more sensors of the electronic device 200. In some implementations, the server 203 can comprise and/or be in communication with an electronic medical records (EMR). An EMR can comprise a propriety EMR. An EMR can comprise an EMR associated with a hospital. An EMR can store data including medical records.

Electronic device 200 can communicate with one or more computing devices 201 via the network 204. Computing devices 201 can include one or more of a patient monitor (for example, a bedside monitor), a patient monitoring and connectivity hub, a handheld patient monitoring device, a wearable patient monitoring device, a mobile communication device (for example, a smartphone), a smart watch, a wearable device, an earbud, a computer (which can be a laptop or a desktop), a tablet, a nurses' station system, physiological sensors, glucose monitors, a home monitoring system, or the like. In some implementations, the computing device(s) 201 can include a same or similar device as the electronic device 200. The electronic device 200 can, via the network 204, communicate data to the computing device(s) 201 and/or receive data from the computing device(s) 201, such as sensor data which can include physiological data. In some implementations, the electronic device 200 can communicate with the computing device(s) 201 directly such as via a wireless and/or wired communication protocol.

The electronic device 200 can communicate directly with the computing device(s) 201 and/or server(s) 203 via a wireless communication protocol including one or more of WiFi, Bluetooth, near field communication (NFC), radio frequency identification (RFID), cellular telephony, Zigbee, Z-wave, and/or the like.

In some implementations, the electronic device 200 can communicate with the server(s) and/or computing devices 201 during a non-privacy mode. In some implementations, the electronic device 200 may not communicate with the server(s) and/or computing devices 201 during a privacy mode. For example, the electronic device 200 may not be capable of communicating with the server(s) and/or computing devices 201 during a privacy mode because a communication component of the electronic device 200 may be powered off.

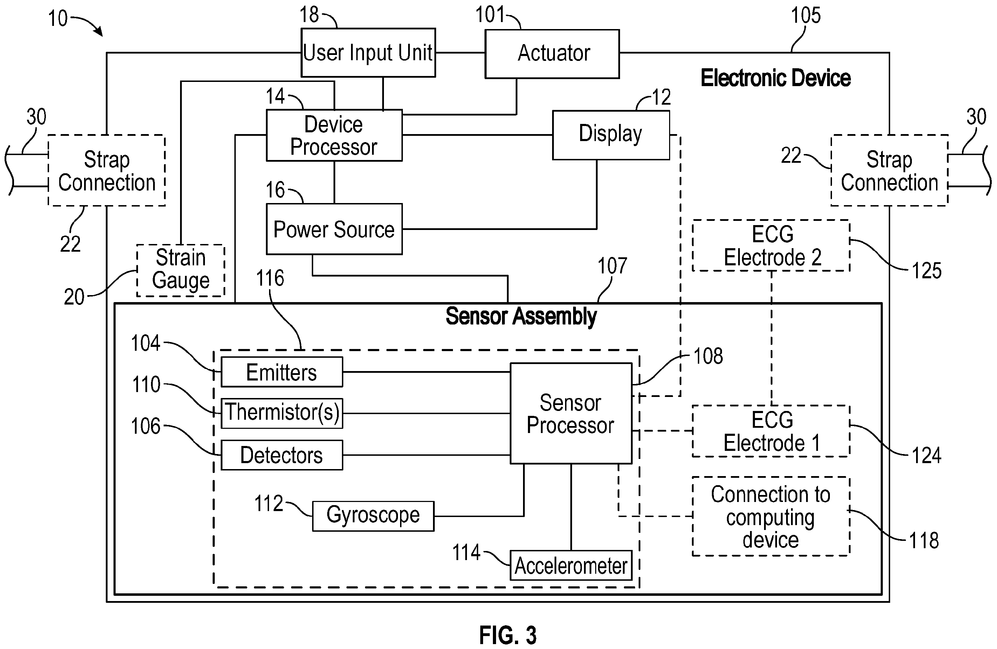

As illustrated in FIG. 3, the electronic device 10 can include a housing 105 coupled to a strap connection 22 and a strap 30. The housing 105 may include a sensor assembly 107 having a printed circuit board 116 (referred to herein as "PCB 116"), a connector 118 to a computing device (referred to herein as "connector 118"), and/or ECG electrodes 124, 125. The PCB 116 may include emitters 104, detectors 106, a sensor processor 108, thermistor(s) 110, a gyroscope 112, and/or an accelerometer 114. The housing 105 may further include a display screen 12, a device processor 14, a power source 16, a user input unit 18, and a strain gauge 20. The electronic device 10 can further include strap 30 and strap connection 22.

The one or more components of the sensor assembly 107, the PCB 116 and/or the wearable electronic device 10 can be in electrical communication with one or more components. As an example and not meant to be limiting, the power source 16 may provide power for components of the PCB 116, the device processor 14, and the display screen 12. The PCB 116 may be in electrical communication with the display screen 12, the user input unit 18, the device processor 14 and/or the like.

A display screen 12 can be positioned at a top side of the device housing 105. The display screen 12 can provide, among other indications, an indication of time and date. The display screen 12 can further include a display layout (for example, the default display layout) indicating the wearer's SpO2 measurement, pulse rate (PR) measurement, respiration rate (RR) measurement, hydration status (H2O), and/or the like. The format of the display layout is not limiting. For example, some measurements, such as the SpO2 measurement and/or PR measurements, can be displayed as numerical values. As another example, some measurements, such as the RR measurements and hydration status, can be displayed as a sliding scale. As an illustrative example, the display screen 12 can indicate a hydration status having three levels from low (L) to high (H). A respiration rate can be displayed as ranging from 5 bpm to 25 bpm. The display screen 12 may allow the wearer to transition between user interfaces similar to and/or the same as described with reference to the user input unit 18. For example, the display screen 12 may be a touch screen allowing a user to view individual display layouts for each measurement or a group of measurements, transition and/or display additional measurements within a user interface, and/or the like, by one or more of and/or a combination of a tap, a press, a slide, a touch, a pinch, a swipe, and/or the like on the display screen 12. The display screen 12 may provide one or more measurements as described herein. Each of the measurements can be displayed constantly, at certain intervals, and/or upon receiving instructions for display (for example, by the wearer tapping on the display screen 12, sliding the user input unit 18, and/or pressing a button on the electronic device 10).

The display screen 12 can display measurements with different or the same frequencies. Time and certain physiological parameters (for example, SpO2 and pulse rate) can be immediately and/or intermittently available, and/or continuously measured (for example, at least every 5 to 10 measurements per minute or more) and the displayed values constantly updated. Optionally, the display screen 12 can further show a trend line for some parameters, such as SpO2 and pulse rate. In one example, the display of the wearable electronic device 10 can be configured to display only time, SpO2, and pulse rate.

In some examples, the display screen 12 can use e-ink or ULP (ultra-low power screen) technology, which draws little amount of current for displaying information. The display screen 12 may automatically adjust the brightness, being brighter when outdoors and dimmer when indoors to further prolong battery life.

A device processor 14 can be a digital/analog chip or other processor(s), such as a digital watch processor or a smartwatch processor. The electronic device 10 may include nonvolatile memory to store the critical patient information and/or computer-executable instructions that may be executed by the device processor 14. The device processor 14 can execute one or more functions of the electronic device 10 as described herein. For example, the device processor 14 can generate an instruction of the display to continue displaying critical patient information (for example, the patient's name, date of admission, etc.) after the power source 16 has been depleted. In other examples, the device processor 14 can process a reading from a strain gauge 20 and transmit an instruction to display the processed reading of the pressure asserted by the electronic device 10 on the wearer to the display screen 12. Further, the device processor 14 may, in response to determining a change in state of the user input unit 18 (e.g., via a single tap, a double tap, a short tap, a long tap, a slide, a press, and/or the like), may generate user interface data and display user interface data via display screen 12. In some examples, the processor 14 may, in response to a change in state of the user input unit 18, scroll through one or more user interfaces which can include various indicia of measurements and/or display layouts of the display screen 12.

Power source 16 can include a battery for powering one or more components of the electronic device 10 such as the device processor 14, the display screen 12, and/or the sensor assembly 107. The battery 16 can last at least 10 hours, or at least 12 hours, or at least 14 hours, or at least about 16 hours after each charge, with continuous measurements and/or displaying of certain physiological parameters, such as SpO2 and pulse rate. In some examples, the power source 16 can send an indication to the display screen 12, after the power source 16 has been depleted, even if other features (for example, measuring physiological parameters using the sensor assembly 107) may not be available when the power source 16 has been depleted. Additionally, when the electronic device 10 is used clinically, the display screen 12 can also continue displaying critical patient information (for example, the patient's name, date of admission, etc.) after the battery 16 has been depleted.

As described above, the electronic device 10 can include a user input unit 18. The user input unit 18 may sense an input by a user, such as one or more of and/or a combination of: a tap, a press, a slide, a touch, a pinch, a swipe, and/or the like. The user input unit 18 may include one or more and/or a combination of different types of user input unit 18 such as, but not limited to capacitive, resistive, infrared, surface capacitive, and/or projected capacitive touch (PCAP) devices.

A capacitive user input unit 18 may detect a user's capacitance by registering changes in capacitance when, for example, a finger of a user and/or a stylus interacts with the user input unit 18. A resistive user input unit 18 may include multiple layers (e.g., two flexible layer sheets coated with a resistive material and/or the like) where pressing the screen causes the layers to touch, generating a signal. An infrared user input unit 18 may include an array of infrared LEDs and photodetectors around the parameter of a user input unit 18, to detect an interruption in infrared light beams when touched. A surface capacitive user input unit 18 may function similarly to a capacitive user input unit 18, however the surface capacitive user input unit 18 may operate with a single of electrodes placed on a glass surface of the user input unit 18, enabling better durability and/or quality. Additionally, a user input unit 18 may be a PCAP device, where a grid of electrodes detect touch, allowing for multi-touch functionality (e.g., a pinch, two simultaneous presses, and/ the like) and higher sensitivity than one or more other types of user input units 18.

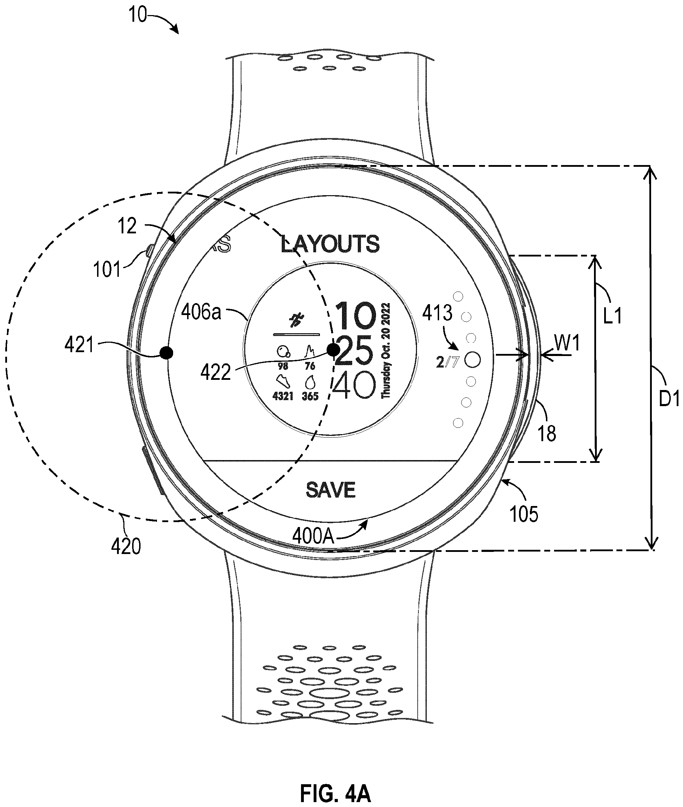

User input unit 18 may be electrically coupled to the device processor 14, sensor processor 108, display screen 12, and/or the like. The user input unit 18 may receive an input by a user in the form of, but not limited to, a tap, a press, a slide, a touch, a pinch, a swipe, and/or the like, on the user input unit 18. For example, and as further discussed with reference to FIGS. 4A-4C, a user input unit 18 may cause a display screen 12 to scroll through one or more display layout previews, transition display layouts, manipulate one or more characteristics and/or parameters associated with a display layout, and/or the like. In some examples, device processor 14 may monitor a state of the user input unit 18 and, in response to a determined state of the user input unit 18, generate signals to cause a display unit 12 to updates its user interfaces as described herein. Additionally and/or alternatively, the user input unit 18 may be used by the device processor 14 to change and/or control another feature other than a display. For example, the user input unit 18 may receive an input by a user resulting in an adjustment of a volume output level from the electronic device 10, a brightness level of the display unit 12, a zoom level of user interfaces displayed within display unit 12, an adjustment to a tactile feedback setting (e.g., a vibration and/or the like), or another non-display function as described herein. In some implementations, the user input unit 18 may receive a fingerprint of a user to identify the user (and to authorize the user to use the electronic device 10 and/or perform operations with the electronic device 10).

The electronic device 10 can include an optional strain gauge 20. The strain gauge 20 can measure a pressure of the electronic device 10 on the wearer. Readings from the strain gauge 20 can be communicated to the device processor 14, which can process the readings and output an indication of the pressure asserted by the electronic device 10 on the wearer to be displayed on the display screen 12. In one example, the display screen 12 can display a green light when the pressure on the wearer is suitable for using the sensor assembly 107 and display a red or other colored light for a pressure that is too high or too low than the desired pressure or pressure range. In some examples, once the strain gauge 20 indicates that the desired pressure or pressure range has been achieved, the sensor processor 108 may activate one or more measurements as described herein (e.g., PR, SpO2, and/or the like). Optionally, the device processor 14 can also deactivate the sensor assembly 107, and/or any other sensors on or attached to the electronic device 10, in response to not detecting any readings from the strain gauge 20, indicating that the electronic device 10 is not worn on the wearer.

The strap(s) 30 can be connected to the electronic device 10 using any suitable strap connections 22. For example, the strap connections 22 can be compatible with third party watch bands, wearable blood pressure monitors, and/or the like.

The sensor assembly 107 of the wearable electronic device 10 can include a sensor or sensor processor 108 (which can include a memory, other electronics, and/or the like). The sensor processor 108 can process signals from one or more of the sensors in sensor assembly 107 (or optionally other sensors in communication with the electronic device 10) to determine a plurality of physiological parameters. Processing of raw sensor data of the sensors in communication (via a wired and/or wireless connection) with the sensor or sensor processor 108 may be performed by the sensor or sensor processor 108. For example, the sensor processor 108 can be configured to drive the emitters 104 to emit light of different wavelengths and/or to process signals of attenuated light after absorption by the body tissue of the wearer from the detectors 106.

The sensor processor 108 can determine and output for display on the display screen 12, one or more physiological parameters based on the detected signals. Optionally, the device processor 14 can receive signals from detectors 106 (for example, preprocessed signals) and determine and output physiological parameters for display.

The sensor assembly 107 can include more than one group or cluster of optical emitters 104 (such as LEDs) and more than one group of photodetectors 106 (also referred to as "detectors 106"). Each group of emitters 104 can be configured to emit four (or three) different wavelengths based on. Emitters 104 can be driven by the processor 108, to emit light of four (and/or three) different wavelengths. Detectors 106 may transmit a received reflection of one or more light signals to the processor 108, to determine physiological parameters.

The emitters 341 of the sensor assembly 340 can be configured to emit a plurality of (for example, three, four, five, or more) wavelengths. The emitters 341 can be configured to emit light of a first wavelength providing an intensity signal that can act as a reference signal. The first wavelength can be more absorbent by the human body than light of other wavelengths emitted by the emitters 341. The reference signal can be used by the sensor assembly processor 348 to extract information from the other signals, for example, information relevant to and/or indicative of the pulsing rate, harmonics, or otherwise. The sensor assembly processor 348 can focus the analysis on the extracted information for calculating the physiological parameters of the wearer. The first wavelength can be between about 500 nm and about 540 nm, between about 505 nm and about 535 nm, between about 510 nm and about 530 nm, between about 515 nm and about 525 nm, or about 520 nm, or any value or range between any of these values or ranges or any value or range bounded by any combination of these values, although values or ranges outside these values or ranges can be used in some cases. The light providing the reference signal can have a green color or alternatively an orange color or yellow color.

The emitters 341 can be configured to emit light of a second wavelength having a red or orange color. The second wavelength can be between about 600 nm and about 640 nm, between about 605 nm and about 635 nm, between about 610 nm and about 630 nm, between about 615 nm and about 625 nm, or about 620 nm, or any value or range between any of these values or ranges or any value or range bounded by any combination of these values, although values or ranges outside these values or ranges can be used in some cases. Light of the second wavelength can be more sensitive to changes in SpO2. The second wavelength can be closer to 620 nm (for example, about 625 nm or 620 nm), which results in greater absorption by the body tissue of the wearer, and therefore a stronger signal and/or a steeper curve in the signal, than a wavelength that is closer to 660 nm. The sensor assembly processor 348 can extract information such as the pleth waveform from signals of the second wavelength.

The emitters 341 can be configured to emit light of a third wavelength between about 640 nm and about 680 nm, between about 645 nm and about 675 nm, between about 650 nm and about 670 nm, between about 655 nm and about 665 nm, or about 660 nm, or any value or range between any of these values or ranges or any value or range bounded by any combination of these values, although values or ranges outside these values or ranges can be used in some cases.

The emitters 341 can be configured to emit light of a fourth wavelength between about 900 nm and about 910 nm, between about 900 nm and about 905 nm, between about 905 nm and about 910 nm, or about 905 nm, or about 907 nm, or any value or range between any of these values or ranges or any value or range bounded by any combination of these values, although values or ranges outside these values or ranges can be used in some cases. The fourth wavelength can be in the infrared range. The pulse oximeter processor can use the fourth wavelength as a normalizing wavelength when calculating ratios of the intensity signals of the other wavelengths, for example, a ratio of the intensity signals of the second wavelength (red) to the third wavelength (infrared).

Additionally or optionally, the emitters 341 can be configured to emit light having a fifth wavelength that is more sensitive to changes in water than the other emitted wavelengths. The fifth wavelength can be in the infrared range or between about 950 nm and about 990 nm, between about 955 nm and about 985 nm, between about 960 nm and about 980 nm, between about 965 nm and about 975 nm, or about 970 nm, or any value or range between any of these values or ranges or any value or range bounded by any combination of these values, although values or ranges outside these values or ranges can be used in some cases. The sensor assembly processor 348 can determine physiological parameters such as a hydration status of the wearer based at least in part on a comparison of the intensity signals of the fifth wavelength and a different wavelength detected by certain detectors 345.

The sensor assembly 107 can include one or more thermistors 110 and/or other types of temperature sensors (e.g., RTD, thermocouple, and/or the like). The thermistor(s) 110 can be placed near one or more groups of emitters 104. There can be at least one thermistor 110 near each group of emitters 104. The thermistor(s) 110 can provide for wavelength correction of the light emitted by the emitters 104. Optionally, the thermistor(s) 110 can additionally measure a temperature of the wearer of the electronic device 10. Optionally there can be one or more thermistors 110 located at other places of sensor assembly 107. In some examples, the emitters 104, the thermistor(s) 110, and/or the detectors 106 can be positioned on the PCB 116.

The electronic device 10 can include a gyroscope 112, an accelerometer 114, and/or other inertial sensor. The gyroscope 112 and/or the accelerometer 114 can be in electrical communication with the sensor processor 108. The sensor processor 108 can determine motion information from signals from the gyroscope 112 and/or the accelerometer 114. The motion information can provide a noise reference for analysis of the plethysmograph information and/or other signal processing (for example, processing of ECG signals) performed by the sensor processor 108.

The sensor assembly 107 can include a connector 118 for connecting to a computing device such as a sensor, which can be a plethysmograph sensor or other suitable sensors. The connector 118 can be oriented such that the second sensor can extend from a housing of the electronic device 10 with reduced and/or no impingement of the tissue at the device/tissue interface, resulting in less or no effect of the connector 118 on the blood flow through the device measurement site.

Optionally, the electronic device 10 can include ECG electrodes 124, 125 configured to make contact with the wearer's skin. One or more ECG electrodes 124 may be located on sensor assembly 107. One or more ECG electrodes 125 may be located elsewhere on the electronic device 10 (for example, an ECG electrode 125 can form a part of the housing 105 of the wearable electronic device 10. The ECG electrodes 124, 125 can be in electrical communication with the sensor processor 108 via an ECG connector. The sensor assembly 107 can optionally include a plurality of ECG electrodes 124, 125. Some of the ECG electrodes 125 can be located away from the sensor assembly 107 and some of the ECG electrodes 124 can be located on the sensor assembly 107.

The ECG electrodes 124, 125 can include a negative electrode, a positive electrode, and a reference electrode. Electrodes 124 located on sensor assembly 107 can act as a reference electrode and a negative (or positive) electrode respectively. In some examples, a portion of the housing 105 that surrounds the display screen 12 can function as another ECG electrode 125. In some examples, an electrically insulating material can separate the ECG electrode 125 from the remainder of the housing 105 so that an electrical current between the ECG electrode 125 and the ECG electrodes 124 would travel through the wearer's body. When the wearer wants to make a measurement using the ECG sensor that includes the ECG electrodes 124, 125, the wearer can press on or touch the electrode 125 using the wearer's finger or another part of the wearer's body such that the wearer's skin makes contact with the electrode 125.

FIGS. 4A-4C depict example user interfaces 400A-400C respectively, from a display screen 12 of electronic device 10. User interfaces 400A-400C can occupy an entirety of display screen 12 in some cases or can occupy less than an entirety of display screen 12 in some cases. In other words, the area of the user interfaces 400A-400C can be less than or equal to the area of the display screen 12, depending on the implementation. The example user interfaces 400A-400C illustrate various example functionality of the electronic device 10 as described, for example, in reference to an input from a user on a user input unit 18. Advantageously, the electronic device 10 can enable a user to quickly scroll and/or manipulate user interfaces displayed via a display screen 12 in accordance with one or more inputs by the user on the user input unit 18. FIGS. 4A-4C illustrate an example of using the user input unit 18 to transition between user interfaces 400A-400C to change display layout previews (e.g., 406a-406b) displayed via display screen 12. In some implementations, the user input unit 18 can cause the display screen 12 to transition between other user interfaces such as user interfaces comprising various formats of indicia of physiological parameters, various watch face layouts, various physiological parameter layouts, etc. Accordingly, a user may scroll through and/or select various user interfaces with user input unit 18. In some implementations, the user input unit 18 can cause the display screen 12 to transition between various color schemes associated with user interfaces. For example, a user may be able to transition through various color scheme options with user input unit 18 and select a color scheme with user input unit 18. In some implementations, the user input unit 18 can cause the display screen 12 to change brightness level.

FIG. 4A shows the length L1 and width W1 of the user input unit 18 and the diameter D1 of the display screen 12 (which in some cases may also be equal to the diameter of the user interface 400A). User input unit 18 may have a width W1 such that user input unit 18 protrudes out from the housing 105. As shown here, width W1 varies along the length L1 such that width W1 is thickest at the center of the user input unit 18. In some implementations, width W1 is uniform along the length L1. The width W1, such as at the maximum, can be between 0.05cm and 1.0cm, between 0.10cm and 0.75cm, between 0.10cm and 0.5cm, between 0.10cm and 0.25cm, between 0.10cm and 0.20cm, or between 0.15cm and 0.25cm. The width W1 can be less than the height H1, such as between about 20% and 50% of H1, or any value therebetween. The width W1 of user input unit 18 can provide structural variation from housing 105 to help a user tactilely distinguish between the user input unit 18 and the housing 105.

In this example, D1 represents a diameter because the display is circular 12. In some implementations, D1 may represent a distance between the top of the display screen 12 to the bottom of the display screen 12 such as if the display were rectangular. As discussed in FIG. 1F, L1 can be between about 25% and about 100% of D1, between about 25% and 75% of D1, between about 50% and about 100% of D1, between about 30% and about 70% of D1, between about 40% and about 60% of D1, or any value therebetween. In some implementations, L1 may be between about 10% and about 50% the circumference of the display screen 12, between about 10% and about 25% the circumference of the display screen 12, between about 5% and about 15% the circumference of the display screen 12, between about 25% and about 50% the circumference of the display screen 12, etc. Advantageously, because the size and/or shape of the user input unit 18 may generally correspond with the size and/or shape of the display screen 12, user interaction with the user input unit 12 may more closely correspond with resulting actions on the display screen 12. FIG. 4A may be drawn to scale.

FIG. 4A depicts an example user interface 400A showing display layout preview 406a for user selection. A user can transition through various display layout previews (e.g., 406a, 406b) to select which display layout they would like. The various display layout previews 406a, 460b each correspond with, and are smaller versions of, respect display layouts that govern how elements of a user interface, such as indicia of physiological data, are arranged within the user interface. Path 420 is shown as superimposed on the electronic device 10 and may not be displayed within user interface 400A and/or a physical component of electronic device 10. Path 420 governs how the display layout preview 406a moves within user interface 400A. In this example, path 420 is an arc of a circle with center 421. In this example, center 421 is positioned on electronic device 10, within display screen 12, and on the edge of user interface 400A. In some implementations, center 421 can be positioned entirely outside of user interface 400A, display screen 12, and/or electronic device 10. In some implementations, center 421 can be positioned entirely within user interface 400A. In this example, the diameter of path 420 is equal, or substantially equal to, the diameter of the user interface 400A. In some implementations, the diameter of path 420 can be the same size as diameter D1 of the display screen 12 (which in some cases may be the same size as the diameter of user interface 400A). In some implementations, the diameter of path 420 may be less than the diameter of user interface 400A.

Path 420 can pass through the center of display screen 12. Thus, path center 421 is offset from the center of the display screen 12. In this example, path center 421 is positioned to the left of the center of the display screen 12 such that the center of the display screen 12 is positioned between path center 421 and user input unit 18. Path center 421 can be positioned at any angular position within the 360 degrees surrounding the center of the display screen 12. For example, path 420 can at least partially surround user input unit 18 and path center 421 can be positioned between the center of the display screen 12 and the user input unit 18.

Display layout preview 406a has point 422 which may be located at the center of display layout preview 406a and/or the center of display screen 12. Point 422 lies on path 420 and may be constrained to reside on path 420 as display layout preview 406a moves within user interface 400A. Accordingly, display layout preview 406a (or center thereof) may travel along a path 420 within user interface 400A.

User interface 400A includes indicator 413. As illustrated, the indicator 413 may provide a user with a reference and/or location of a display layout preview 406a on the display screen 12. Indicator 413 comprises a plurality of circles including a larger circle in between smaller circles above and below the larger circle. Indicator 413 is positioned within the display screen 12 on a side proximate the user input unit 18. In some implementations, indicator 413 can be positioned within the display screen 12 across from the user input unit 18. Indicator 413 has the same general shape as the user input unit 18 (e.g., a curve). As depicted in the example user interface 400A, the user is currently viewing the second of seven display layout previews (e.g., display layout preview 406a), however, this is not meant to be limiting as there can be less and/or more display layout previews.

Display layout preview 406a (and/or another display layout preview of the electronic device 10) may include characteristics and/or parameters associated with one or more inputs and/or sensors of the electronic device 10, such as oxygen saturation (SpO2), pulse rate, a plethysmograph waveform, perfusion index (PI), pleth variability index (PVI), methemoglobin (MetHb), carboxyhemoglobin (CoHb), total hemoglobin (tHb), respiration rate, glucose, date and time, a step counter, and/or the like. A display layout preview 406a (and/or any other display layout preview) may be configured by a device processor 14 and/or sensor processor 108.

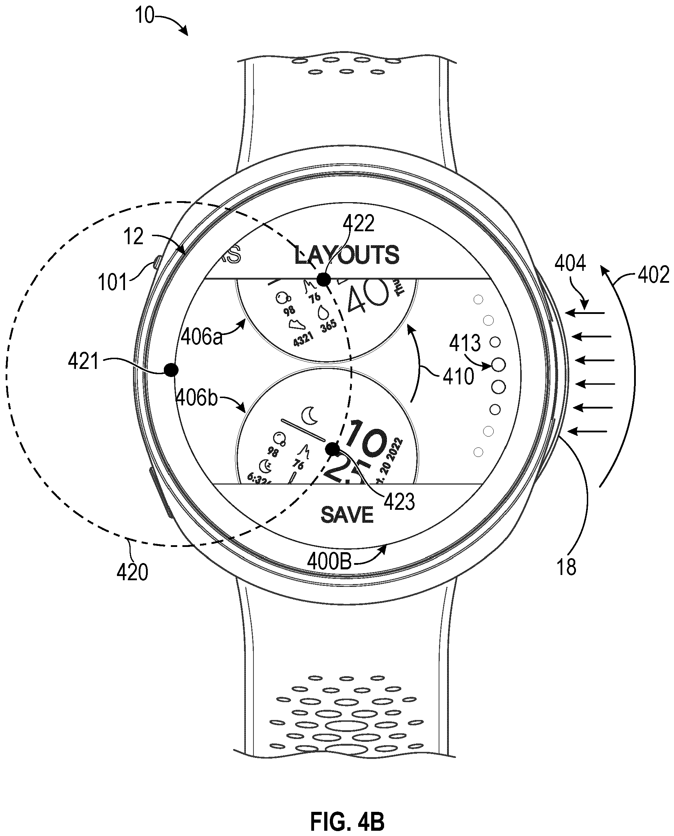

As depicted in example user interface 400B of FIG. 4B, display screen 12 may transition between user interfaces as indicated by arrow 410, from display layout preview 406a to display layout preview 406b. The display screen 12 may transition in response to actuation of user input unit 18. For example, sensor processor 108 can generate a signal (responsive to actuation of user input unit 18) to update display screen 12 with various user interfaces. Actuation of user input unit 18 can include a tap, a press, a touch, (e.g., a single tap, a double tap, a short tap, a long tap, and/or the like) which may be used in some implementations to make selections. Actuation of user input unit 18 can include a slide, a pinch, a swipe, along arrows 402, 404 which may be used in some implementations to transition through various options (e.g., various UI options).

In the example of FIG. 4B, the user has swiped along user input unit 18 in the direction of arrow 402. Based on the user input (e.g., a swipe across the user input unit 18), the device processor 14 and/or sensor processor 108 may determine a change in state of the user input unit 18. Based on the change in state of the user input unit 18, the processor 14 and/or 108 may cause display screen 12 to update user interfaces displayed thereon such as by generating user interface data for rendering and/or updated user interfaces. For example, the processor 14 and/or processor 108 may transmit a signal to the display screen 12, to scroll from display layout preview 406a to display layout preview 406b as indicated by arrow 410. (Note that arrow 410 is superimposed on the example user interfaces 400A-400C and is shown for illustrative purposes only. Arrow 410 does not form part of the example user interface 400A-400C). Additionally and/or optionally, indicator 413 may transition a portion of the user interface 400B by enlarging a next circle in a sequence of circles and reducing the size of a current circle.

The direction in which display layout previews 406 move through display screen 12 may correspond to the direction in which a user slides along user input unit 18. For example, in this case, a user sliding upward along user input unit 18 as shown by arrow 402 can cause display layout previews 406 to also move upward. Likewise, sliding downward along user input unit 18 as shown by arrow 402 can cause display layout previews 406 to move downward. In some cases the display layout previews 406 may move opposite the direction of user input on the user input unit 18. For example, swiping downward on user input unit 18 can cause display layout previews 406 to move upward, and swiping upward can cause display layout previews 406 to move downward. In such an implementation, the path center 421 may be positioned between the center of the display screen 12 and the user input unit 18 such that the path 420 surrounds the user input unit 18.

Movement along the user input unit 18 can result in movement of indicia on the display screen 12 as shown and/or described in FIG. 4B where a user moves their finger along user input unit 18 causing display layout previews 406a and 406b to move along display screen 12. The ratio of user movement on user input unit 18 to movement of indicia on the display screen 12 can be 1:2, where if the user moves their finger certain distance along user input unit 18, indicia on the display screen 12 moves twice as far as that distance. In some implementations, the ratio can be 1:4, 1:3, 1:2.5, 1:1.5, 1:1, 4:1, 3:1, 2.5:1, 2:1, 1.5:1, etc. The ratio can change based on the speed at which the user moves their finger along user input unit 18 with faster movement resulting in a higher output ratio, and vice versa. In some cases, the ratio can also be set by a user. If the ratio were 1:1 and the length L1 of the user input unit 18 were about 50% the diameter D1 of the display screen 12, the user would advantageously only need to move their finger twice along the length L1 of the user input unit 18 to move indicia the entire distance of the diameter D1 of the display screen 12.

Electronic device 10 may be capable of displaying a plurality of unique display layout previews, including display layout previews 406a, 406b. In the examples shown in FIGS. 4A-4C, there may be seven different display layout previews as indicated by indicator 413 showing that display layout preview 406a is the 2^{nd} of 7 options and that display layout preview 406b is the 3^{rd} of 7 options. Electronic device 10 may display less than all of the possible display layout previews simultaneously. For example, in FIG. 4B, display screen 12 shows only two of the seven display layout previews simultaneously. In some implementations, electronic device 10 may display no more than two of the display layout previews simultaneously.

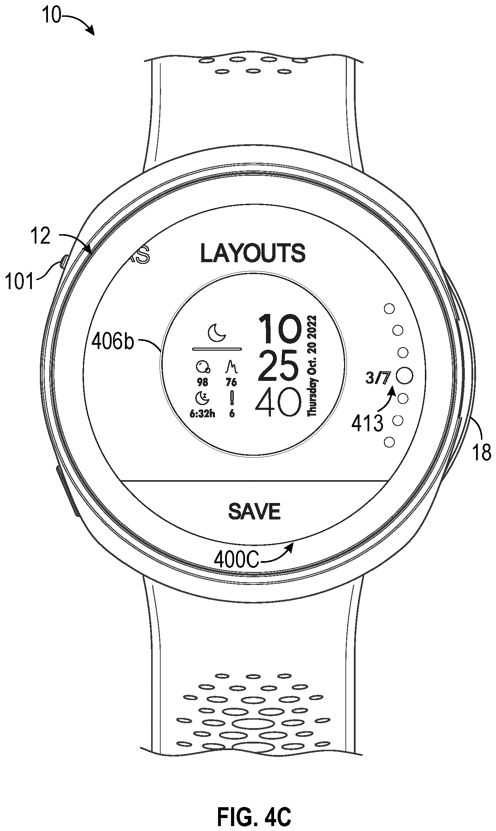

As depicted in example user interface 400C of FIG. 4C, display screen 12 indicates display layout preview 406b in response to an input by a user via user input unit 18. Indicator 413 transitions to indicate that the third of seven layouts is displayed. To show display layout preview 406a (e.g., a previously displayed layout option), a user may swipe in an opposite direction along user input unit 18 and/or may a tap, a press, a slide, a touch, a pinch, a swipe, and/or the like, along the user input unit 18. Additionally and/or alternatively, user input unit 18 may transmit a signal to a device processor 14, sensor processor 108, display screen 12, and/or the like, based on an input from a stylus and/or another component.

A user can select display layout preview 406b (or any of the others) as the option they would like to select for the corresponding display layout to govern the arrangement of elements, such as indicia of physiological data, within the display screen 12. The user can make the selection via the user input unit 18 and/or the display screen 12. For example, a user can touch on the display screen 12 and/or the user input unit 18 to select display layout preview 406b. Processor 14 can determine a change in state on the display screen 12 and/or user input unit 18 responsive to the user's touch and can generate instructions in response to cause the display screen 12 to update based on the selection.

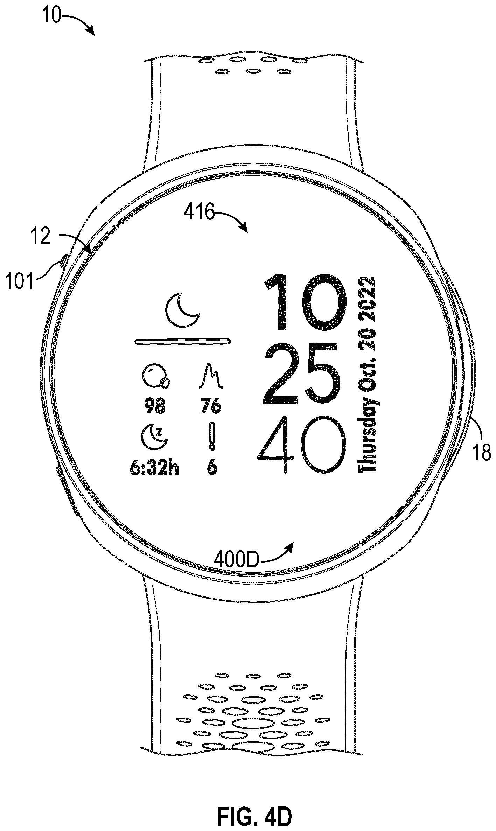

FIG. 4D illustrates electronic device 10 with display screen 12 showing user interface 400D with display layout 416. Display layout 416 corresponds to display layout preview 406b and is displayed within display screen 12 responsive to user selection of display layout preview 406b. Display layout preview 406b may be a smaller rendition of display layout 416. Display layout preview 406b provides a visualization of display layout 416 on a smaller scale of size. The display layout preview 406b can be between 20% and 90% the size of display layout 416, or between 50% and 90%, between 50% and 80%, between 50% and 70%, between 30% and 50%, between 40% and 50%, between 40% and 60%, or any value therebetween. Display layout 416 governs how user interface elements, such as indicia of physiological data, are arranged within user interface 400D. Display layout 416 can occupy an entirety of user interface 400D and/or display screen 12. In some cases, display layout 416 may be used interchangeably with user interface 400D.

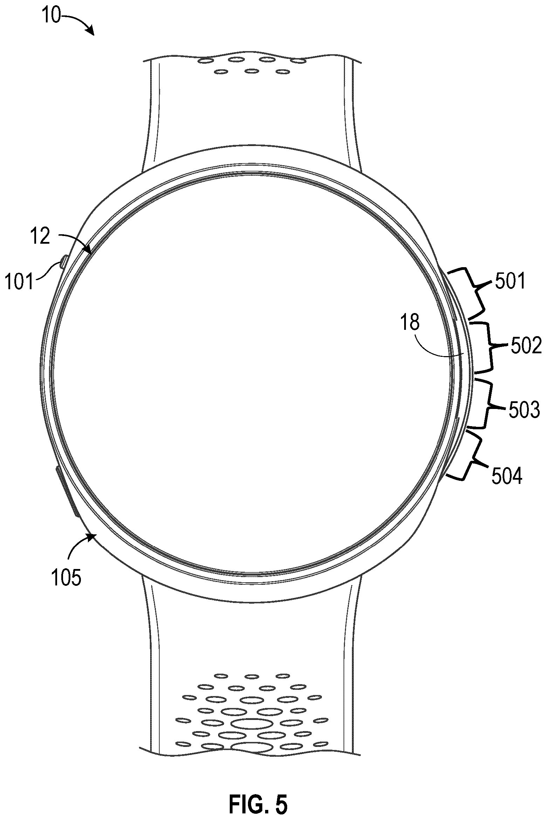

FIG. 5 illustrates a front view of the wearable electronic device 10 with housing 105, display screen 12, and user input unit 18. The user input unit 18 can comprise a plurality of zones, such as first zone 501, second zone 502, third zone 503, and fourth zone 504. Each of the zones 501-504 can be independently responsive to user input (e.g., touch) and can independently communicate with an internal processor. For example, a processor of the electronic device 10 can register user input from zone 501 independently of registering user input from the other zones 502-504. The user input unit 18 can have less than four zones, such as three zones, two zones, or one zone. The user input unit 18 can have more than four zones, such as five zones, six zones, seven zones, or more than seven zones.

A hardware processor of the electronic device 10 can monitor the states of the zones 501-504 to determine one or more characteristics of a user input, such as direction of a user input motion, speed of a user input motion, location along the user input unit 18 were a user touches, time that a user touches the user input unit 18 or its various zones, etc.

For example, if a user swipes their finger along the user input unit from bottom to top, starting at zone 504 and ending at zone 501, the processor can determine that the user has swiped their finger in an upward direction and can control one or more operations accordingly, such as to transition user interfaces in an upward direction (illustrated in FIGS. 4A-4C), or to increase a volume of a speaker output, or to increase a brightness of display screen 12, etc. Thus, the processor can determine a direction in which the user has swiped based on determining an order in which the user interacts with the various zones 501-504.

As another example, if a user sequentially touches more than one of the zones 501-504 (e.g., while swiping along user input unit 18 with their finger), the processor can determine a speed with which the user transitions between contacting the various zones to determine how fast the user is swiping along the user input unit 18 and can control one or more operations accordingly, such as to transition between user interfaces more rapidly or slowly for a faster or slower swipe, respectively or to transition through a greater or smaller number of user interfaces for a faster or slower swipe, respectively. The processor can also vary the speed at which it changes volume output or display screen 12 brightness based on swipe speed. Thus, the processor can determine the speed that a user swipes along user input unit 18 based on determining the amount of time between user interactions with the various zones 501-504.

As another example, if a user touches a single zone, the processor can determine where on the user input unit 18 the user has touched and can control one or more operations based on the location of the touch, such as to increase volume output to a set amount based on the zone (e.g., 100% output volume if zone 501 is touched by itself or 0% output volume if zone 504 is touched by itself). Corresponding controls could also be implemented for selecting user interfaces to display via the display screen 12, for selecting selectable components within user interfaces, or for controlling brightness of the display screen 12.

The zone 501-504 may not be marked on the user input unit 18. For example, a user may not be able to distinguish the various zones 501-504 based on touch or sight, etc. The user may be unaware of the existence of the zones 501-504. In some implementations, the zones 501-504 may be demarcated on the user input unit 18 for a user to distinguish such as visually and/or tactilely. Zones that are adjacent may contact each other. A user can contact more than one zone at a time.

One or more of the zones 501-504 can be a same size as each other or may be a different size from each other. Implementing one more of the zones 501-504 with a different size can allow for varying degrees of control along the user input unit 18. For example, a user may wish to implement control with a high degree of precision via a top portion of the user input unit 18 such that zones 501-502 can be smaller than zones 503-504 thus allowing for finer granularity of touch via zones 501-502. Correspondingly, a user may wish to implement control with a lower degree of precision via a bottom portion of the user input unit 18 such that zones 503-504 can be larger than zones 501-502 thus allowing for larger ranges of motion to via zones 503-504 to implement certain operations.

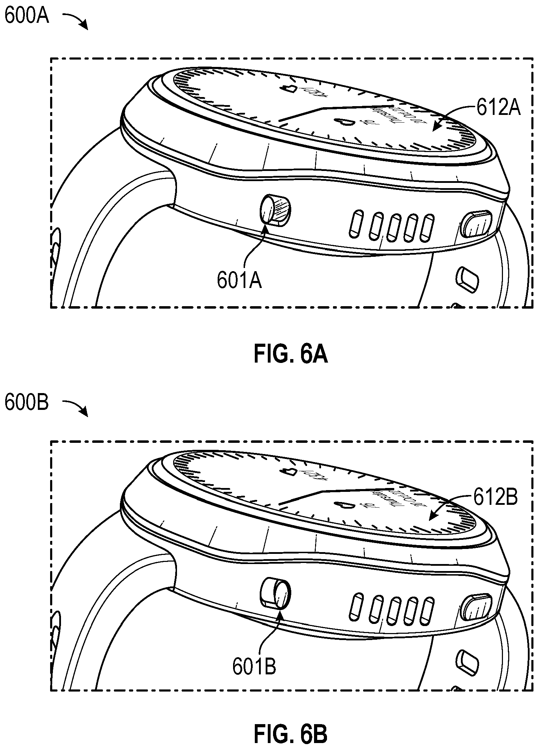

FIG. 6A is a perspective view of an example implementation of an electronic device 600A. The electronic device 600A can include an actuator 601A and a display 612A. The display 612A can be configured to display data, including sensor data obtained by one or more sensors of the electronic device 600A.

The actuator 601A can be a slideable switch. The actuator 601A can be configured to transition between states. For example, the actuator 601A can slide between a first state and a second state. The actuator 601A can cause the electronic device 600A to transition between privacy and non-privacy modes of operation. For example, a user can actuate the actuator 601A from one state to another to cause the electronic device to enter a privacy mode or a non-privacy mode.

The actuator 601A, or portion thereof, can include shading or coloring. For example, a portion of the actuator 601A can be colored red, green, black, or any other color, and can be viewable when the actuator 601A is in a certain state. In the example implementation shown in FIG. 6A, the actuator 601A is in a certain state which can cause the electronic device 600A to enter a privacy mode. A portion of the actuator 601A can be visible and can be colored a certain color, such as green, black, red, etc., to indicate the electronic device 600A is in the privacy mode.

In some implementations, the actuator 601A can be configured to transition between two states which can correspond to a privacy mode and a non-privacy mode. In some implementations, the actuator 601A can be configured to transition between more than two states. For example, 601A can be configured to transition between three states which can correspond to a privacy mode, a first privacy mode wherein the sensors are powered on and continue to operate and one or more privacy components, such as a communication component, is powered off and does not operate, and a second privacy mode wherein one or more sensors and one or more privacy components are powered off and do not operate.

During a privacy mode, the display 612A can be configured to display data such as physiological data obtained from one or more sensors. The display 612A can be configured to display data that was generated and/or obtained during a privacy mode. Advantageously, a user of the electronic device 600A can be able to continue to view data via the display 612A during a privacy mode, while the data is still protected by the electronic device 600A. In some implementations, the display 612A may not display data during a privacy mode, for example, because the display 612A can be powered off during the privacy mode.

FIG. 6B is a perspective view of an example implementation of an electronic device 600B. The electronic device 600B can include an actuator 601B and a display 612B. The electronic device 600A, or its components, such as the actuator 601B and/or display 612B can include similar structural and/or operational features as electronic device 600A shown and/or discussed with respect to FIG. 6A.

The actuator 601B, or portion thereof, can include shading or coloring. For example, a portion of the actuator 601B can be colored red, green, black, or any other color, and can be viewable when the actuator 601B is in a certain state. In the example implementation shown in FIG. 6B, the actuator 601B is in a certain state which can cause the electronic device 600B to enter a non-privacy mode. A portion of the actuator 601B can be visible and can be colored a certain color, such as green, black, red, etc., to indicate the electronic device 600B is in the non-privacy mode. In some implementations, the portion of the actuator 601B that is visible in FIG. 6B when the actuator 601B is in one state can be different than the portion of the actuator 601A that is visible in FIG. 6A when the actuator 601A is in another state.

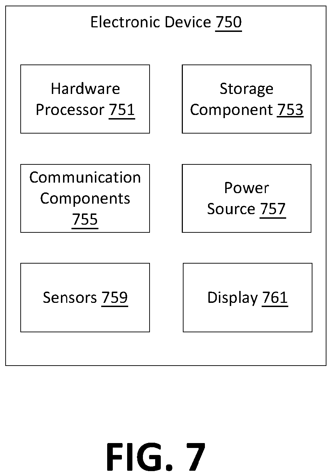

FIG. 7 is a block diagram of an example electronic device 750. Electronic device 750 can include similar structural and/or operational features as any of the other example electronic devices shown and/or described herein. Electronic device 750 can be a wearable device such as a watch, a soundbar, a phone such as a mobile phone, a monitoring hub, a physiological sensor, a tablet computer, desktop computer, laptop, mobile computing device, mobile phone, personal digital assistant (PDA), hybrid PDA/mobile phone, or the like. Electronic device 750 can include a hardware processor 751, a storage component 753, one or more communication components 755, a power source 757, one or more sensors 759, and a display 761.

The hardware processor 751 can comprise one or more integrated circuits. The hardware processor 751 can comprise and/or have access to memory. The hardware processor 751 can comprise and/or be embodied as one or more chips, controllers such as microcontrollers (MCUs), and/or microprocessors (MPUs). The hardware processor 751 can comprise a central processing unit (CPU). In some implementations, the hardware processor 751 can be embodied as a system-on-a-chip (SoC). The hardware processor 751 can be configured to implement an operating system which can allow multiple processes to execute simultaneously. The hardware processor 751 can be configured to execute program instructions to cause the electronic device 750, or components thereof, to perform one or more operations. The hardware processor 751 can be configured, among other things, to process data, execute instructions to perform one or more functions, and/or control the operation of the electronic device 750 or components thereof. For example, the hardware processor 751 can process physiological data obtained from physiological sensors and can execute instructions to perform functions related to storing and/or transmitting such physiological data. In some implementations, the hardware processor 751 may be remote to the electronic device 750.

The storage component 753 can include any computer readable storage medium and/or device (or collection of data storage mediums and/or devices), including, but not limited to, one or more memory devices that store data, including without limitation, dynamic and/or static random-access memory (RAM), programmable read-only memory (PROM), erasable programmable read-only memory (EPROM), electrically erasable programmable read-only memory (EEPROM), optical disks (e.g., CD-ROM, DVD-ROM, etc.), magnetic disks (e.g., hard disks, floppy disks, etc.), memory circuits (e.g., solid state drives, random-access memory (RAM), etc.), and/or the like. Such stored data can be processed and/or unprocessed physiological data originating from physiological sensors, for example. The storage component 753 can store program instructions that when executed by the hardware processor 751 cause the electronic device 750 to perform one or more operations.

The communication components 755 can facilitate communication (via wireless, wired, and/or wire-like connection) between the electronic device 750 (and/or components thereof) and separate devices, such as separate electronic devices, monitoring devices, computing devices, mobile devices, sensors, systems, servers, or the like. For example, the communication components 755 can be configured to allow the electronic device 750 to wirelessly communicate with other devices, systems, and/or networks over any of a variety of communication protocols, including near-field communication protocols and far-field communication protocols. Near-field communication protocols, which may also be referred to as non-radiative communication, can implement inductive coupling between coils of wire to transfer energy via magnetic fields (e.g., NFMI). Near-field communication protocols can implement capacitive coupling between conductive electrodes to transfer energy via electric fields. Far-field communication protocols, which may also be referred to as radiative communication, can transfer energy via electromagnetic radiation (e.g., radio waves). The communication components 755 can communicate via any variety of communication protocols such as Wi-Fi, Bluetooth^{®}, ZigBee^{®}, Z-wave^{®}, cellular telephony, such as long-term evolution (LTE) and/or 1G, 2G, 3G, 4G, 5G, etc., infrared, radio frequency identification (RFID), satellite transmission, inductive coupling, capacitive coupling, proprietary protocols, combinations of the same, and the like. In some implementations, communication components 755 can implement human body communication (HBC) which may include capacitively coupling a transmitter and receiver via an electric field propagating through the human body. The communication components 755 can allow data and/or instructions to be transmitted and/or received to and/or from the electronic device 750 and separate computing devices. The communication components 755 can be configured to transmit and/or receive (for example, wirelessly) processed and/or unprocessed physiological data with separate computing devices including physiological sensors, other monitoring hubs, remote servers, or the like. In some implementations, communication components 755 can transfer power required for operation of a computing device. The communication components 755 can be embodied in one or more components that are in communication with each other. The communication components 755 can include one or more of: transceivers, antennas, transponders, radios, emitters, detectors, coils of wire (e.g., for inductive coupling), and/or electrodes (e.g., for capacitive coupling). The communication components 755 can include one or more integrated circuits, chips, controllers, processors, or the like, such as a Wi-Fi chip, a Bluetooth chip, and/or a cellular telephony chip and/or modem.

The power source 757 can provide power for components of the electronic device 750. The power source 757 can include a battery. In some implementations, the power source 757 can be external to the electronic device 750. For example, the auricular device 200 can include or can be configured to connect to a cable which can itself connect to an external power source to provide power to the electronic device 750. The power source 757 can include a dual-battery configuration with a main battery and a backup battery. The electronic device 750 can additionally or alternatively be configured to be solar-powered, for example, by including a solar panel on the dial or elsewhere of the electronic device 750.

The sensors 759 can include one or more physiological sensors configured to generate physiological data of physiological parameters. The sensors 759 can include acoustic sensors, optical sensors, inertial sensors, temperatures sensors, electrical sensors, voltage sensors, impedance sensors, etc. The sensors 759 can include an oximeter. The sensors 309 can include a photoplethysmography (PPG) sensor configured to measure volumetric variation in blood circulation and derive one or more parameters therefrom, such as pulse rate, blood pressure, respiration rate, cardiac output, perfusion index, pleth variability index, PPG waveform data, blood oxygen saturation, etc. The sensors 309 can include one or more optical emitters configured to emit optical radiation of a plurality of wavelengths, which can include visible light. The sensors 309 can include one or more optical detectors configured to detect optical radiation attenuated by the tissue of subject (which may have been emitted by optical emitters) and generate data relating to the pulsatile characteristics of the subject, including blood oxygen saturation, hydration, hemoglobin content, etc. The sensors 759 can include electrocardiogram (ECG) sensors, including one or more electrodes, configured to measure electrical activity of the subject, such as cardiac signals. The sensors 759 can measure and/or generate data relating to respiration rate, blood oxygen saturation (e.g., SpO2), heart rate, pulse rate, skin temperature, core body temperature, spatial orientation, or the like.

The sensors 759 can include a microphone configured to generate audio data responsive to detecting audio. The sensors 759 can include a camera configured to generate image data responsive to capturing light. Image data generated by the camera can be processed to render images and/or video.

The display 761 can display user interfaces, such as any of the example user interfaces, or aspects thereof, that are shown and/or described herein. The display 761 can include an LED screen, an LCD screen, an OLED screen (such as an AMOLED screen), a QLED screen, a plasma display screen, a quantum dot display screen, or the like. The display 761 may be responsive to touch. For example, the display screen can comprise a touchscreen such as a resistive touchscreen, a capacitive touchscreen, an infrared touchscreen, a surface acoustic wave touchscreen, or the like.

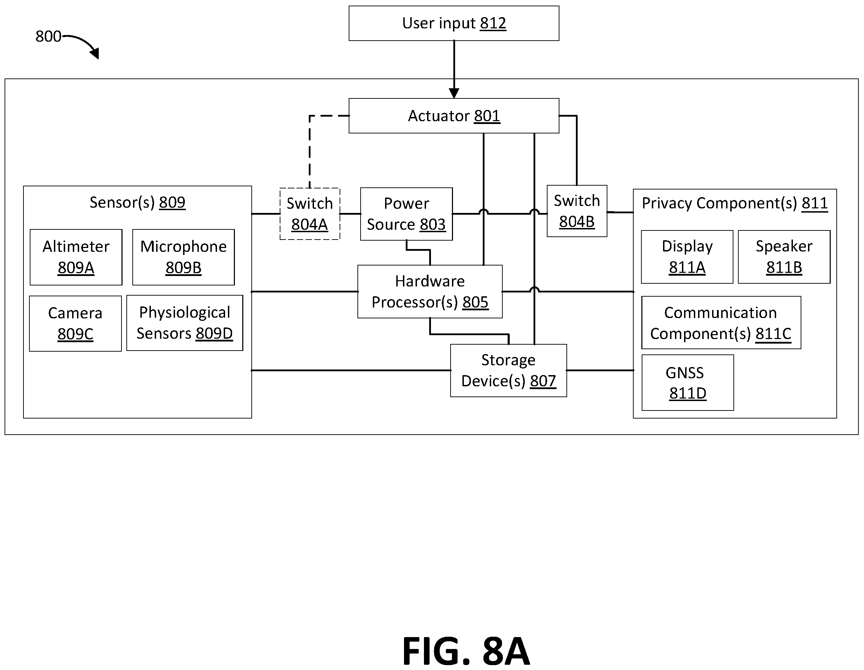

FIG. 8A is a schematic block diagram illustrating an example implementation of an electronic device 800. Electronic device 800 can be implemented as any of the other example electronic devices shown and/or described herein. The electronic device 800 can include an actuator 801, one or more sensors 809, one or more privacy components 811, a power source 803, one or more hardware processors 805, one or more storage devices 807, and one or more switches such as switch 804A and/or switch 804B. In some implementations, the electronic device 800 may not include switch 804A.

The power source 803 can provide power to hardware components of the electronic device 800 described herein. The power source 803 can include a battery. The power source 803 can be, for example, a lithium battery. Additionally or alternatively, the electronic device 800 can be configured to obtain power from a power source that is external to the electronic device 800. For example, the electronic device 800 can include or can be configured to connect to a cable which can itself connect to an external power source to provide power to the electronic device 800.

The switch(es) 804 (such as switch 804B and/or switch 804A) can include an electrical switch, relay, conductor, circuit breaker, or the like. The switch(es) 804 can include a two-way, three-way, four-way switch, or the like. The switch(es) 804 can include a single pole, single throw (SPST) switch, a single pole, double throw (SPDT) switch, a double pole, single throw (DPST) switch, a double pole, double throw (DPDT) switch, or the like. The switch(es) 804 can be configured to transition between one or more states, such as in response to input from the actuator 801. In some implementations, the switch(es) 804 can transition between one or more states in response to a signal from the processor(s) 805. In some implementations, the switch(es) 804 can transition between states automatically based, at least in part, on one or more conditions such as time, location of the electronic device 800, or the like. The switch(es) 804 can be configured to conduct energy in a first state and not conduct energy in a second state. The switch 804B can be configured to electrically disconnect the privacy component(s) 811 from the power source 803. The switch 804A can be configured to electrically disconnect the sensor(s) 809 from the power source 803. In some implementations, the switch 804A may be different than switch 804B. In some implementations, switch 804A may be the same as switch 804B.

The processor(s) 805 can be configured, among other things, to process data, execute instructions to perform one or more functions, and/or control the operation of the electronic device 800, or components thereof. For example, the processor(s) 805 can process data obtained from the sensor(s) 809 and can execute instructions to perform functions related to processing, storing, displaying, and/or transmitting such data.

The storage device(s) 807 can include one or more memory devices that store data, including without limitation, dynamic and/or static random-access memory (RAM), programmable read-only memory (PROM), erasable programmable read-only memory (EPROM), electrically erasable programmable read-only memory (EEPROM), and the like. Such stored data can be data obtained from the sensor(s) 809 which can include processed and/or unprocessed physiological data, for example.

The sensor(s) 809 can include an altimeter 809A, a microphone 809B, a camera 809C, and/or one or more physiological sensors 809D. FIG. 8A is not intended to be limiting of the present disclosure. In some implementations, the sensor(s) 809 can include additional sensors not shown. In some implementations, the sensor(s) 809 can include fewer sensors than what are shown.

The sensor(s) 809 can generate sensor data, for example in response to obtaining, measuring, and/or collecting input data. The altimeter 809A can generate altitude data in response to obtaining barometric pressure data. The microphone 809B can generate audio data in response to receiving audio input. The camera 809C can generate image and/or video data in response to capturing image input. The physiological sensor(s) can generate physiological data in response to obtaining physiological measurements of user of the electronic device 800. The physiological sensor(s) 809D can include one or more of electrodes, emitters, detectors, thermistor, gyroscope, accelerometer, or the like.

The privacy component(s) 811 can include a display 811A, a speaker 811B, one or more communication components 811C, and/or a GNSS 811D. FIG. 8A is not intended to be limiting of the present disclosure. In some implementations, the privacy component(s) 811 can include additional devices not shown. In some implementations, the privacy component(s) 811 can include fewer devices than what are shown. In some implementations, the privacy component(s) 811 may not include a display 811A and/or a speaker 811B.

The display 811A can display one or more images relating to sensor data. For example, the display 811A can display physiological parameters or data obtained from one or more physiological sensors 809D. The display 811A can include an LED display.

The speaker 811B can output auditory signals. The auditory signals can include an alarm. The auditory signals can relate to sensor data such as data from physiological sensors 809D. The auditory signals can include sounds and/or verbal words.

The GNSS 811D can facilitate communication between the electronic device 800 (and/or components thereof) and separate devices such as one or more satellites. The GNSS can transmit and/or receive data relating to a geographic location of the GNSS 811D and electronic device 800. The GNSS 811D can include a transceiver, an antenna, or the like. In some implementations, the GNSS 811D can be embodied in the communication component 811C.

The communication component(s) 811C can facilitate communication (via wired and/or wireless connection) between the electronic device 800 (and/or components thereof) and separate devices, such as separate computing devices, sensors, systems, servers, or the like. For example, the communication component(s) 811C can be configured to allow the electronic device 800 to wirelessly communicate with other devices, systems, and/or networks over any of a variety of communication protocols. The communication component(s) 811C can be configured to use any of a variety of wireless communication protocols, such as Wi-Fi, Bluetooth^{®}, ZigBee^{®}, Z-wave^{®}, cellular telephony, infrared, near-field communications (NFC), radio frequency identification (RFID), satellite transmission, proprietary protocols, combinations of the same, and/or the like. The communication component(s) 811C can allow data and/or instructions to be transmitted and/or received to and/or from the electronic device 800 and separate computing devices. The communication component(s) 811C can be configured to transmit and/or receive (for example, wirelessly) sensor data such as processed and/or unprocessed physiological data with separate computing devices remote to the electronic device 800, remote servers, or the like. The communication component(s) 11C can be embodied in one or more components that are in communication with each other. The communication component(s) 811C can include a wireless transceiver, an antenna, and/or a near field communication (NFC) component such as a transponder.

The power source 803 can be in electrical communication with the privacy component(s) 811 via switch 804B. The power source 803 can be removably electrically coupled with the privacy component(s) 811 via the switch 804B. For example, the switch 804B can electrically connect the power source 803 with the privacy component(s) 811 in a first state and can electrically disconnect the power source 803 from the privacy component(s) 811 in a second state. The switch 804B can transition between states in response to actuation of the actuator 801.

Transitioning of the switch 804B between states can correspond to various modes of operation of the electronic device 800. For example, the switch 804B can electrically disconnect the power source 803 from the privacy component(s) 811 during a privacy mode operation and can electrically connect the power source 803 to the privacy component(s) 811 during a non-privacy mode operation. One or more of the privacy component(s) 811 may not operate when disconnected from the power source 803 by the switch 804B, such as during a privacy mode. Advantageoulsy, when the privacy component(s) 811 are electrically disconnected from the power source 803 by the switch 804B, such as during a privacy mode, the electronic device can protect private data, such as sensitive sensor data, by preventing the data from being transmitted, obtained, received, viewed, heard, or the like, by other computing devices, people, etc., for example. For example, during a privacy mode, when the privacy component(s) 811 may be electrically disconnected from the power source 803, the communication component(s) 811C may not be capable of communicating data to one or more remote devices at least because the communication component(s) 811C are powered off. As another example, during a privacy mode, the communication component(s) 811C may not be capable of receiving data, such as phone calls, text messages, email messages, software updates, notifications, or the like, from one or more remote devices at least because the communication component(s) 811C are powered off. As another example, during a privacy mode, display 811A may be disabled. For example, the display 811A may not display data, such as physiological data, at least because the display 811A is powered off, which can prevent certain people from viewing the data on the display 811A such as when a user of the electronic device 800A is in a public area and does not desire other people to view data displayed via the display 811A. As another example, during a privacy mode, the speaker 811B may not be capable of outputting an audio signal, at least because the speaker 811B is powered off, which can prevent certain people from hearing audio signals relating to private data such as when a user of the electronic device 800 is in a public area and does not desire other people to hear audio sounds relating to certain data, such as voice audio reciting physiological parameters of the user. As another example, during a privacy mode, the GNSS 811D may not be capable of determining a location of the GNSS 811D and/or the electronic device 800, at least because the GNSS 811D is powered off, which can prevent other computing devices and/or people from receiving, obtaining data relating to the location of the GNSS 811D and/or the electronic device 800.

In some implementations, the switch 804B can transition between states to electrically connect/disconnect each of the privacy component(s) 811 collectively from the power source 803. For example, the switch 804B can electrically connect/disconnect the power source 803 with each of the privacy component(s) 811 via a single pole. In some implementations, the switch 804B can transition between states to electrically connect/disconnect the privacy component(s) 811 from the power source 803 independently of one another. For example, the switch 804B can electrically connect/disconnect some or all of the privacy component(s) 811 with the power source 803 individually such as via multiple switches or multiples poles. For example, each pole can correspond to a unique device of the privacy component(s) 811.

As an example, the switch 804B can electrically connect each of the privacy component(s) 811 with the power source 803 in a first state and can electrically disconnect each of the privacy component(s) 811 from the power source 803 in a second state. As another example, the switch 804B can electrically connect a first group of privacy component(s) 811 with the power source 803 in a first state and can electrically connect a second group of privacy component(s) 811 with the power source 803 in a second state and can electrically connect each of the privacy component(s) 811 with the power source 803 in a third state and can electrically disconnect each of the privacy component(s) 811 from the power source 803 in a fourth state.

In some implementations, the power source 803 can be in electrical communication with the privacy component(s) 811 optionally via switch 804B. The power source 803 can be removably electrically coupled with the privacy component(s) 811 via the switch 804B. For example, the switch 804B can electrically connect the power source 803 with the privacy component(s) 811 in a first state and can electrically disconnect the power source 803 from the privacy component(s) 811 in a second state. The switch 804B can transition between states in response to actuation of the actuator 801. The switch 804A can include similar and/or the same structural and/or operational features discussed with respect to switch 804B.

In some implementations, the power source 803 can be in direct electrical communication with the privacy component(s) 811. For example, the power source 803 may not be electrically coupled with the privacy component(s) 811 via the switch 804A. For example, the sensor(s) 809 can be in electrical communication with the power source 803 and can receive power therefrom whether during a privacy mode or non-privacy mode. Advantageously, the sensor(s) 809 can continue to operate during a privacy mode of the electronic device 800. In some implementations, the sensor(s) 809 can continue to operate regardless of actuation of the actuator 801. Advantageously, the sensor can continue to generate sensor data whether during a privacy mode or non-privacy mode such that the sensor data may be continuous over a length and/or may not include any gaps due to changes in modes of operation. Advantageously, a continuous set of data may be important and can improve data monitoring and/or analysis, such as when analyzing trends in data, as is often the case with physiological data, for example, for which a user may desire to have access to an uninterrupted series of data. Advantageously, the electronic device 800 may still protect private sensor data that is generated during a privacy mode such as by preventing the sensor data from being communicated to any external devices, for example, at least because the communication component 811C is powered off during a privacy mode. Advantageously, a user of the electronic device 800 may still have access to all sensor data, including private sensor data, generated during a privacy mode. For example, the sensor(s) 809 can generate sensor data during a privacy mode and the display 811A can display that sensor data during and/or after the privacy mode such that a user of the electronic device 800 can view the private sensor data. However, the electronic device 800 may still protect such private sensor data by preventing the communication component 811C from communicating the sensor data to remote devices during and/or after the privacy mode, for example. Accordingly, the electronic device 800 can continue to generate sensor data during a privacy mode while still protecting that sensor data.

The electronic device 800 can receive a user input 812, such as via the actuator 801. The user input 812 can include a physical motion or movement such as a mechanical force to push, slide, switch, toggle, move, etc. the actuator 801. The user input 812 can include an electrical force or signal, such as a change in capacitance on a capacitive touchscreen, a change in voltage, a change in current, or the like. The user input 812 can include one or more auditory signals, such as a voice command detected by a microphone and processed by a processor (for example, using voice recognition), to actuate the actuator 801. The user input 812 can include one or more visual signals, such as an image of a face captured by a camera and processed by a processor (for example, using face recognition), to actuate the actuator 801.

The actuator 801 can change states based on the user input 812. Actuation of the actuator 801 can cause the switch 804A and/or the switch 804B to change states, which can cause the electronic device 800 to transition between one or more operating modes, such as privacy and non-privacy modes. The actuator 801 may be directly physically and/or electrically connected with the switch 804A and/or the switch 804B. For example, the actuator 801 may be hardwired with the switch 804A and/or the switch 804B. Advantageously, a direct connection between the actuator 801 and the switch(es) 804 may ensure that actuation of the actuator 801 causes the switch(es) 804 to change state. Advantageously, transitioning the switch(es) 804 between states may depend only on actuating the actuator 801 (which may be a physical action), and may not depend on program instructions, such as instructions generated by a hardware processor, which may reduce the risk of erroneous operation caused by software bugs, software hacking, software incompatibilities, or the like.

The actuator 801 is in communication with the processor(s) 805. The actuator 801, or actuation thereof, can cause the processor(s) 805 to perform one or more operations and/or change mode of operation. For example, the processor(s) 805 can process data differently based on actuation of the actuator 801.

The processor(s) 805 can receive sensor data from the sensor(s) 809. The processor(s) 805 can tag sensor data with metadata. The metadata can include identifiers to specify one or more attributes of the sensor data, such as a time the sensor data was generated, a mode of operation (for example, privacy, non-privacy) during which the sensor data was generated, a location the sensor data was generated, a privacy status level of the sensor data, or the like. The 805 can assign and/or update the metadata of the sensor data based at least on actuation of the actuator 801. Advantageously, metadata of the sensor data can improve efficiency with which the data is handled and improve protection of private data. For example, the processor(s) 805 can perform one or more operations relating to the sensor data according to metadata of the sensor data. The processor(s) 805 can handle certain sensor data, such as private data, differently based on its metadata. The processor(s) 805 may be able to quickly and efficiently identify data that should be handled differently based on the metadata, which may lead to faster search times when accessing and/or retrieving data such as from storage.

The processor(s) 805 can store sensor data in the storage device(s) 807. In some implementations, the processor(s) 805 can store all sensor data received from the sensor(s) 809. For example, the processor(s) 805 may store sensor data regardless of whether the sensor data was received from and/or generated by the sensor(s) 809 during a privacy mode. In some implementations, the processor(s) 805 may not delete sensor data received from and/or generated by the sensor(s) 809 during a privacy mode. Advantageously, sensor data, whether received and/or generated during a privacy mode, may be stored in the storage device(s) 807 and thus the electronic device 800 may have access to all of the sensor data, while still protecting private data as described herein. This may reduce the amount data that is lost and/or deleted for being private but which may still be valuable and desirable to be kept, although being private and requiring protection. In some implementations, the processor(s) 805 may delete and/or may not save to storage sensor data received from and/or generated by the sensor(s) 809 during a privacy mode.

The processor(s) 805 can store all of the sensor data in a same storage device and/or similar location in storage. For example, the storage device(s) 807 can include a single storage device configured to store all of the sensor data. In some implementations, the processor(s) 805 can store all of the sensor data in a similar manner and/or location, such as regardless of whether the sensor data was received and/or generated during a privacy mode, for example. Advantageously, the processor(s) 805 can identify, retrieve, and/or access sensor data from storage based on metadata of the sensor data, and not based on sensor data location in storage. Advantageously, by identifying its metadata, the processor(s) 805 may be able to more quickly access the sensor data, at least because the processor(s) 805 will not have to search across multiple storage locations and/or devices. Advantageously, the processor(s) 805 may not be required to expend time and processing power to allocate storage, partition storage, segregate storage, etc. to store private data differently than other data. Advantageously, the entire storage device(s) 807 may be available to store any data (for example, private or non-private) which may improve memory requirements, such as by reducing the need to allocate a relatively large amount of storage space for a relatively small amount of data such as private data. Advantageously, eliminating the need to allocate particular storage for certain data, such as private data, may reduce the amount of storage space required by the electronic device 800.

Advantageously, the processor(s) 805 and/or storage device(s) 807 may be in direct communication with the sensor(s) 809. For example, the storage device(s) 807 and/or storage device(s) 807 may be in communication with the sensor(s) 809 regardless of whether the electronic device 800 is in a privacy mode or non-privacy mode and/or whether the actuator 801 is actuated.

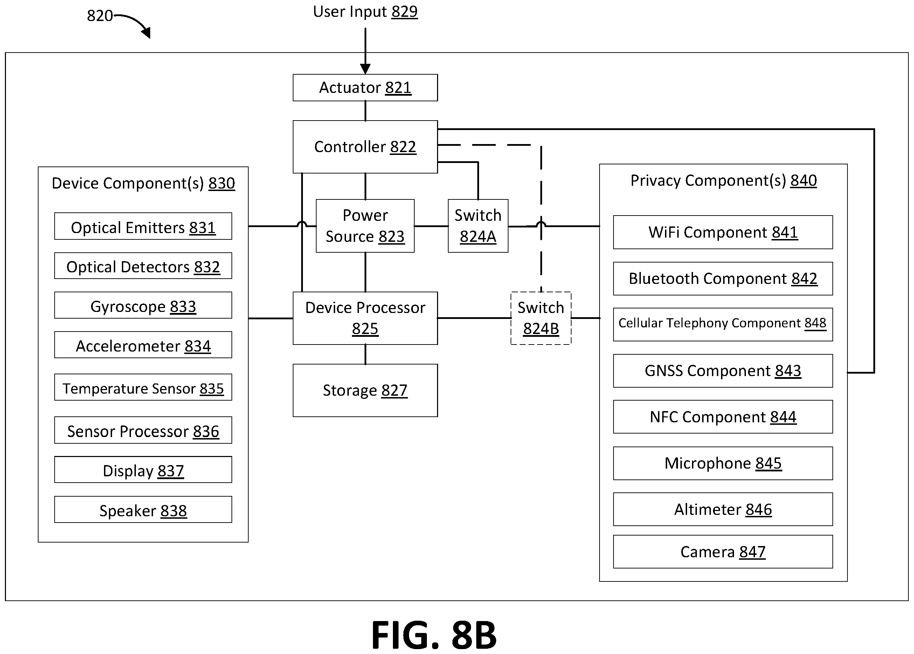

FIG. 8B is a block diagram of an example electronic device 820. The electronic device 820 can include similar structural and/or operational features as any of the other example electronic devices shown and/or described herein. The electronic device 820 may be a phone, a watch, a computer, a soundbar, or the like. Electronic device 820 can include an actuator 821, a controller 822, power source 823, a device processor 825, storage 827, one or more switches (e.g., switch 824A and/or switch 824B), device components 830, and privacy components 840.

The privacy components 840 can include WiFi component 841, a Bluetooth component 842, a cellular telephony component 848, a Global Navigation Satellite System (GNSS) component 843, NFC component 844, microphone 845, altimeter 846, and camera 847. The privacy components 840 may be the components that are eligible to be disabled during a privacy mode. The privacy components 840 can include one or more communication components, such as WiFi component 841, a Bluetooth component 842, a GNSS component 843, and NFC component 844, which can include similar structural and/or operation features as any of the example communication components shown and/or described herein.

The WiFi component 841 can facilitate wireless communication between the electronic device 820 and separate devices, such as separate electronic devices. The WiFi component 841 can implement a WiFi communication protocol to communicate with remote electronic devices (e.g., over a network, which may include the Internet). The WiFi component 841 can allow data and/or instructions to be transmitted from the electronic device 820 (e.g., data from the device processor 825 and/or the storge 827) to separate computing devices. The WiFi component 841 can allow data and/or instructions to be received at the electronic device 820 from separate computing devices, which the WiFi component 841 can communicate to the device processor 825, such as for processing and/or storage. The WiFi component 841 can be embodied in one or more components that are in communication with each other. The WiFi component 841 can include one or more transceivers, antennas, transponders, and/or radios. The WiFi component 841 can include one or more integrated circuits, chips, controllers, processors, or the like, such as a WiFi chip, which may be configured to execute instructions to perform operations and which may be in communication with the controller 822 and/or the device processor 825.

The Bluetooth component 842 can facilitate wireless communication between the electronic device 820 and separate devices, such as separate electronic devices. The Bluetooth component 842 can implement a Bluetooth communication protocol to communicate with remote electronic devices. The Bluetooth component 842 can allow data and/or instructions to be transmitted from the electronic device 820 (e.g., data from the device processor 825 and/or the storge 827) to separate computing devices. The Bluetooth component 842 can allow data and/or instructions to be received at the electronic device 820 from separate computing devices, which the Bluetooth component 842 can communicate to the device processor 825, such as for processing and/or storage. The Bluetooth component 842 can be embodied in one or more components that are in communication with each other. The Bluetooth component 842 can include one or more transceivers, antennas, transponders, and/or radios. The Bluetooth component 842 can include one or more integrated circuits, chips, controllers, processors, or the like, such as a Bluetooth chip, which may be configured to execute instructions to perform operations and which may be in communication with the controller 822 and/or the device processor 825.

The cellular telephony component 848 can facilitate wireless communication between the electronic device 820 and separate electronic devices, such as base transceiver stations and/or mobile devices. The cellular telephony component 848 can implement a cellular communication protocol, such as 1G, 2G, 3G, 4G, 5G, and/or LTE, to communicate with remote electronic devices (e.g., over a network, which can include a cellular network). The cellular telephony component 848 can allow data and/or instructions to be transmitted from the electronic device 820 (e.g., data from the device processor 825 and/or the storge 827) to separate computing devices. The cellular telephony component 848 can allow data and/or instructions to be received at the electronic device 820 from separate computing devices, which the cellular telephony component 848 can communicate to the device processor 825, such as for processing and/or storage. The cellular telephony component 848 can implement voice calls over a cellular network. The cellular telephony component 848 can be embodied in one or more components that are in communication with each other. The cellular telephony component 848 can include one or more transceivers, antennas, transponders, and/or radios. The cellular telephony component 848 can include one or more integrated circuits, chips, modems, controllers, processors, or the like, which may be configured to execute instructions to perform operations and which may be in communication with the controller 822 and/or the device processor 825.

The GNSS component 843 can facilitate wireless communication between the electronic device 820 and remote satellites in orbit around the Earth. The GNSS component 843 can allow data and/or instructions to be transmitted and/or received to and/or from the electronic device 820 and satellites. For example, the GNSS component 843 can receive data from one or more satellites and can determine a location of the GNSS component 843 (and the electronic device 820) from the data received from the satellites. The GNSS component 843 can communicate data received from satellites and/or location data generated by the GNSS component 843 to the device processor 825 which can process said data and/or store said data in storage 827. The GNSS component 843 can be embodied in one or more components that are in communication with each other. The GNSS component 843 can include one or more transceivers, antennas, transponders, and/or radios. The GNSS component 843 can include one or more integrated circuits, chips, controllers, processors, or the like, which may be configured to execute instructions to perform operations and which may be in communication with the controller 822 and/or the device processor 825. The GNSS component 843 can implement one or more of GPS, GLONASS, Galileo, BDS, IRNSS, NavIC, and/or QZSS.

The NFC component 844 can facilitate communication between the electronic device 820 and separate devices. The NFC component 844 can allow the electronic device 820 to communicate with other devices, systems, and/or networks via near-field communication protocols. Near-field communication protocols, which may also be referred to as non-radiative communication, can implement inductive coupling between coils of wire to transfer energy via magnetic fields (e.g., NFMI). Near-field communication protocols can implement capacitive coupling between conductive electrodes to transfer energy via electric fields. In some implementations, NFC component 844 can implement human body communication (HBC) which may include capacitively coupling a transmitter and receiver via an electric field propagating through the human body. The NFC component 844 can allow data and/or instructions to be transmitted from the electronic device 820 (e.g., data from the device processor 825 and/or the storge 827) to separate computing devices. The NFC component 844 can allow data and/or instructions to be received at the electronic device 820 from separate computing devices, which the NFC component 844 may communicate to the device processor 825, such as for processing and/or storage. The NFC component 844 can be embodied in one or more components that are in communication with each other. The NFC component 844 can include one or more of: emitters, detectors, coils of wire (e.g., for inductive coupling), and/or electrodes (e.g., for capacitive coupling). The NFC component 844 can include one or more integrated circuits, chips, controllers, processors, or the like, which may be configured to execute instructions to perform operations and which may be in communication with the controller 822 and/or the device processor 825.

The microphone 845 can detect audio (e.g., sound). The microphone 845 can generate an electrical signal (e.g., audio data) responsive to detecting audio. The microphone 845 can include a transducer configured to convert sound pressure waves into an electrical signal. The microphone 845 may be in communication with the controller 822 and/or the device processor 825. The microphone 845 can generate and communicate audio data to the device processor 825 which can process said data and/or store said data in storage 827.

The altimeter 846 can measure pressure, such as the pressure of the atmosphere surrounding the electronic device 820. The altimeter 846 can generate pressure data based on pressure measurements. The altimeter 846 can determine altitude of the altimeter 846 (and electronic device 820) from pressure data. The altimeter 846 can communicate pressure data and/or altitude data to the device processor 825 which can process said data and/or store said data in storage 827.

The camera 847 can capture light. The camera 847 can generate image data from captured light. The camera 847 can communicate image data to the device processor 825 which can process said data and/or store said data in storage 827.

The device components 830 can include one or more sensors. The device components 830 can include optical emitters 831, optical detectors 832, a gyroscope 833, an accelerometer 834, a temperature sensor 835, a sensor processor 836, a display 837, and a speaker 838. The device components 830 may be the components that are not eligible to be disabled during a privacy mode. The device components 830, including sensors, can continue to operate regardless of the state of the actuator 821 and/or a privacy mode state.

The optical emitters 831 can include can emit optical radiation having one or more wavelengths, such as three, four, five, or six wavelengths. Optical radiation can include infrared radiation and/or visible light. The optical emitters 831 can include one or more light emitting diodes (LEDs).

The optical detectors 832 can include one or more photodetectors, such as photodiodes. The optical detectors 832 may generate data responsive to detecting optical radiation emitted from the optical emitters 831 (e.g., after attenuation by the tissue of a subject).

The gyroscope 833 can generate motion information, such as rotation data including angular velocity and/or acceleration. The accelerometer 834 can generate motion information, including acceleration data with respect three orthogonal directions.

The temperature sensor 835 can generate temperature data indicating a temperature of a subject. The temperature sensor 835 can include a thermistor.

The sensor processor 836 can control operations of the device components 830. For example, the sensor processor 836 can drive the optical emitters 831 to emit light of different wavelengths. The sensor processor 836 can process signals from one or more of the device components 830. For example, the sensor processor 836 can process signals generated by the detectors 852 responsive to detecting attenuated light after absorption by the body tissue of the user. The sensor processor 836 can generate processed physiological data, including physiological parameters, from sensor data. Physiological parameters can include pulse rate, heart rate, respiration rate, SpO2, Pleth Variability Index (PVI), Perfusion Index (PI), respiration from the pleth (RRp), total hemoglobin (SpHb), hydration, glucose, blood pressure, hydration index, and/or other parameters. In some implementations, the sensor processor 836 can perform some or all of the processing of the raw sensor data of the sensors. The sensor processor 836 can cause sensors to perform intermittent and/or continuous data generation, or can perform a spot check, for example, upon request by the user.

The sensor processor 836 can implement electrocardiography (ECG) with sensor data (e.g., electrical data originating from an electrode) to generate processed physiological data. For example, the sensor processor 836 can implement ECG techniques to determine heart rate, can generate ECG waveform data, and/or can determine cardiac arrythmias from sensor data. The sensor processor 836 can implement photoplethysmography (PPG) to determine volumetric changes in the tissue of a user, such as volumetric changes in arterial blood flow. For example, the sensor processor 836 can implement PPG techniques to determine one or more of pulse rate, blood pressure, respiration rate, cardiac output, perfusion index, pleth variability index, and/or PPG waveform data. The sensor processor 836 determine pulsatile characteristics of the subject from optical detector data such as blood oxygen saturation, hydration, hemoglobin content, etc.

In some implementations, the device processor 825 can perform one or more of the operations described with respect to sensor processor 836. In some implementations, the electronic device 820 may not include a sensor processor 836 and the device processor 825 can perform the operations described for sensor processor 836.

The display 837 can display indicia of physiological data, such as physiological parameters. The display 837 can render user interfaces including physiological data.

The speaker 838 can emit audio such as alerts, notifications, alarms, voice call audio, or the like.

The device processor 825 can comprise one or more integrated circuits. The device processor 825 can comprise and/or have access to memory. The device processor 825 can comprise and/or be embodied as one or more chips, controllers such as microcontrollers (MCUs), and/or microprocessors (MPUs). The device processor 825 can comprise a central processing unit (CPU). In some implementations, the device processor 825 can be embodied as a system-on-a-chip (SoC). The device processor 825 can be configured to implement an operating system which may allow multiple processes to execute simultaneously. The device processor 825 can be configured to execute program instructions to cause the electronic device 820, or components thereof, to perform one or more operations. The device processor 825 can be configured, among other things, to process data, execute instructions to perform one or more functions, and/or control the operation of the electronic device 820 or components thereof. For example, the device processor 825 can process data obtained from the device components 830 and/or the privacy components 840 and can execute instructions to perform functions related to storing and/or transmitting such data. The device processor 825 may be in communication with the device components 830, the privacy components 840, the storage 827, or other components of the electronic device 820. The device processor 825 can comprise program instructions that are programmable (e.g., can be updated, modified, provisioned, etc.). For example, the device processor 825 can receive firmware updates on a periodic basis.

The storage 827 can include any computer readable storage medium and/or device (or collection of data storage mediums and/or devices), including, but not limited to, one or more memory devices that store data, including without limitation, dynamic and/or static random-access memory (RAM), programmable read-only memory (PROM), erasable programmable read-only memory (EPROM), electrically erasable programmable read-only memory (EEPROM), optical disks (e.g., CD-ROM, DVD-ROM, etc.), magnetic disks (e.g., hard disks, floppy disks, etc.), memory circuits (e.g., solid state drives, random-access memory (RAM), etc.), and/or the like. Data stored at the storage 827 can include processed and/or unprocessed physiological data, sensor data, data originating from device components 830, and/or data originating from privacy components 840. The storage 827 can store program instructions that when executed by the device processor 825 cause the electronic device 820 to perform one or more operations.

The actuator 821 can be positioned on a frame or housing of the electronic device 820. The actuator 821 can be accessible and/or operable by a user. The actuator 821 can comprise one or more physical components such as switch, button, lever, slider, pin, knob, dial, or the like. The actuator 821 can exist in one of a plurality of states which can include physical states or electrical states. The electrical states can correspond with, and depend on, the physical states such that causing the actuator 821 to change physical states also cause the actuator 821 to change electrical states. Actuator 821 can include and/or be electrically connected with an electrical output pin which can generate different output (e.g., voltages) based on the state of the actuator 821. The actuator 821 output may be binary (e.g., high or low) or tertiary. Thus, electrical output at an actuator 821 output pin can depend on the state of the actuator 821 and can change whenever the actuator 821 changes states. Thus, the state of the actuator 821 can be monitored by monitoring the electrical output from the actuator 821. In some implementations, actuator 821 can be implemented in software, such as program instructions configured to perform operations responsive to user input via a touchscreen.

The actuator 821 may be pressed, slid, rotated, turned, pulled, switched, etc. The actuator 821 may change states based on user input 829. User input 829 may be a physical input such as a force to physically move the actuator 821. For example, a user may physically move the actuator 821 to cause the actuator 821 to change states. User input 829 can include a voice command detected by the actuator 821 which may include a microphone and which may be configured to implement voice recognition. User input 829 can include a gesture detected by the actuator 821 which may include a camera. The actuator 821 may comprise a component in an interactive graphical user interface (GUI). For example, the actuator 821 can be displayed as a selectable GUI component, such as a slidable or pressable GUI component, within a display screen that is responsive to touch (e.g., a resistive touchscreen, a capacitive touchscreen, an infrared touchscreen, a surface acoustic wave touchscreen) and the user input 829 can include a user interaction with the selectable GUI component via the touch responsive display screen.

The actuator 821 may exist in a state that corresponds to a privacy mode and a state that corresponds to a non-privacy mode. The actuator 821 may exist in one of a plurality of states corresponding to a plurality of respective different types of privacy modes. Types of privacy modes can correspond to the components they disable and/or how they disable components (e.g., by cutting power and/or resetting the components). As an example, the actuator 821 may be a slidable switch and may exist in a left position corresponding to a non-privacy mode, a middle position corresponding to a privacy mode wherein certain components are operational and other components are disabled, and a right position corresponding to a privacy mode wherein all eligible components are disabled. In some implementations, the actuator 821 may exist in only one state at a time.

The controller 822 may comprise a microcontroller unit (MCU). The controller 822 may comprise one or more independent and/or dedicated processors configured to execute program instructions. The controller 822 may comprise one or more integrated circuits. The controller 822 may comprise and/or have access to memory. The controller 822 may also be referred to as a dedicated controller or a privacy controller. The controller 822 may be non-programmable. For example, program instructions stored on, accessible by, and/or executable by the controller 822 may not be programmable, such as by utilizing a ROM memory. The controller 822 may comprise program instructions, which after initial configuration (e.g., during manufacturing) can not be updated, modified, provisioned, etc. The controller 822 may not receive, and/or may not be capable of receiving, firmware updates, such as may be provisioned over-the-air or via a physical connection. Advantageously, because the program instructions of the controller 822 may not change, the operation of the controller 822 may not be susceptible to malware.

The controller 822 may be electrically connected with the actuator 821. The controller 822 can monitor the state of the actuator 821 such as by monitoring an electrical output from the actuator 821 which in some cases can correspond with, and depend on, the physical position of the actuator 821. For example, the controller 822 can determine whether the actuator 821 is in a privacy state or a non-privacy state based on an electrical output from the actuator 821 which can depend on the physical position of the actuator 821. The controller 822 may continuously monitor the state of the actuator 821. In some implementations, the controller 822 may be the only component in communication with, and/or electrically connected to, the actuator 821. For example, the controller 822 may be the only device to read the electrical output from the actuator 821 indicative of the state of the actuator 821. For example, the device processor 825 may not read the state of the actuator 821, in some cases.

The controller 822 may be electrically connected with and/or in communication with the device processor 825. For example, the controller 822 can communicate signals to the device processor 825 and/or can receive signals from the device processor 825. The controller 822 may be a non-isolated controller in that the controller can communicate with the device processor 825 and/or that the operation of the controller 822 may be affected by the device processor 825 or signals communicated therefrom. The controller 822 may be accessible by the device processor 825 and vice, versa. In some implementations, the controller 822 may be in one-way communication with the device processor 825. For example, the controller 822 can communicate data to the device processor 825 but may not receive data from the device processor 825. Accordingly, the controller 822 may be able to control an operation of the device processor 825 but may not be controllable by the device processor 825 in some cases. In such implementations, the controller 822 can implement a privacy mode upon expiration of a time threshold after actuation of actuator 821 without considering whether the device processor 825 has confirmed that the electronic device 820 is prepared for the privacy mode (or without even sending a notification signal to the device processor 825 to prepare for privacy mode).

The controller 822 can communicate a signal (e.g., a notification signal) to the device processor 825 in response to determining that the actuator 821 is in a privacy state. The notification signal can cause the device processor 825 to prepare the electronic device 820 to enter a privacy mode. Implementing a privacy mode on the electronic device 820 can include disabling components, severing power to components, resetting components, etc. which may cause processes that are executing on the electronic device to 820 to terminate. Terminating a process suddenly or without adequate preparation may cause loss of data, such as if the process is terminated asynchronously. The device processor 825 can prepare the electronic device 820 to enter a privacy mode such as by terminating processes executing on the electronic device 820, which can include synchronously terminating the processes, storing data in storage, or the like. The device processor 825 can terminate processes that are executing on the device processor 825, on the sensor processor 836, or on other processors or circuits of the electronic device 820. The device processor 825 can communicate a signal (e.g., a confirmation signal) to the controller 822. The confirmation signal can indicate that the electronic device 820 is ready to safely enter a privacy mode (e.g., that processes executing on the electronic device 820 have been properly terminated). The device processor 825 can communicate the confirmation signal to the controller 822 in response to successfully terminating one or more processes.

The controller 822 can implement a privacy mode on the electronic device 820 in response to one or more conditions. The conditions can include receiving the confirmation signal at the controller 822 from the device processor 825 and/or expiration of a time period. For example, the controller 822 can implement a privacy mode in response to receiving a signal from the device processor 825 indicating that the electronic device 820 is ready to enter a privacy mode. In some implementations, the controller 822 may wait to initiate the privacy mode until receiving confirmation from the device processor 825 that the electronic device 820 is ready to enter the privacy mode. As another example, the controller 822 can initiate a timer in response to determining that the actuator 821 is in a privacy state and/or in response to communicating a notification signal to the device processor 825. The controller 822 can initiate the privacy mode in response to the timer exceeding a threshold. The timer (or threshold associated with the timer) can be between 1 second and 60 seconds, between 1 second and 30 seconds, between 1 second and 10 seconds, between 1 second and 5 seconds, between 1 second and 3 seconds, or between 1 second and 2 seconds. The timer (or threshold associated with the timer) can be between 0.1 seconds and 1 second or between 0.1 seconds and 0.5 seconds. The timer (or threshold associated with the timer) can be about 1 second, about 2 seconds, about 3 seconds, about 4 seconds, or about 5 seconds. As an example, the controller 822 can initiate a privacy mode 5 seconds after determining that the actuator 821 is in a privacy state and/or after communicating the notification signal to the device processor 825. In some implementations, a time between determining that the actuator 821 is in a privacy state and communicating the notification signal may be negligible. The controller 822 can initiate a privacy mode after a time period has elapsed regardless of whether the controller 822 has received a signal from the device processor 825 confirming that the electronic device 820 is ready to safely enter a privacy mode. For example, the controller 822 can initiate a privacy mode after expiration of a time period even if one or more processes are still executing on the electronic device 820. The device processor 825 may fail to prepare the electronic device 820 to enter a privacy mode (e.g., within a certain time frame), in some examples, if malicious software is executing on the device processor 825 and preventing the device processor 825 from operating properly. Advantageously, the controller 822 may bias the decision to initiate privacy mode on the side of privacy such as by prioritizing privacy over safely executing and terminating processes. For example, the controller 822 may initiate a privacy mode to ensure privacy even if doing so may result in data loss or crashing a process that is currently executing.

Other conditions that may cause the controller 822 to implement a privacy mode can include location data or time data. For example, the controller 822 can implement a privacy mode in response to determining that the electronic device 820 is in a certain location or in response to determining that a current date, time, day, etc, match a criteria. For example, the controller 822 can implement a privacy mode at a particular time of day, or certain days of the week. The controller 822 may receive location data or time data from the device processor 825, from another device on the electronic device 820, or from a remote device or server.

The power source 823 can include a battery and/or capacitor, such as lithium-based battery such as a lithium-ion battery. The power source 823 can include an energy generation system, such as a system configured to generate electrical energy from solar energy. The power source 823 can be removably integrated with the electronic device 820. The power source 823 can be rechargeable such as with inductive charging and/or direct electrical contact. The power source 823 may provide power to the electronic device 820 for the electronic device 820 to operate. The power source 823 may provide power to at least the device components 830, the controller 822, the device processor 825, the storage 827, the privacy components 840, among others. The power source 823 may be fixedly coupled to the device components 830 without a switch therebetween such that the device components 830 can be continuously connected to the power source 823 regardless of a privacy mode state. The power source 823 may be selectively coupled to the privacy components 840. For example, the power source 823 may be removably coupled to the privacy components 840 via switch 824A. The power source 823 may selectively provide power to the privacy components 840 based on an operation of the switch 824A. In some implementations, the power source 823 may be coupled to the privacy components 840 via a plurality of switches, such as one switch per privacy component 840.

The switch 824A may comprise one or more switches. The switch 824A may comprise an analog switch. The switch 824A may be embodied in circuitry. The switch 824A may comprise one or more MOSFETs. The switch 824A can transition between a plurality of states, such as an open state and a closed state. The switch 824A may be a single pole, double throw (SPDT) switch. In some implementations, the switch 824A can transition between two or more states which may correspond to a non-privacy mode and one or more different types of privacy modes (for example, two types of privacy can be implement, a first privacy mode disabling all communication components, camera and audio device and a second mode where only the camera and audio devices are disabled, but communication components are not disabled). The switch 824A can be a single pole, triple throw (SPTT) switch, or may include more than three throws. The switch 824A can conduct an electrical signal (comprising data and/or energy) in a closed state. The switch 824A may inhibit the conduction of electrical signals in an open state. The switch 824A may allow the power source 823 to provide power to one or more of the privacy components 840 when the switch 824A is in the closed state. The switch 824A may inhibit the power source 823 from providing power to one or more of the privacy components 840 when the switch 824A is in an open state. The switch 824A can control whether the power source 823 is electrically coupled with one or more privacy components 840. The switch 824A can sever an electrical connection between the power source 823 and one or more privacy components 840.

The controller 822 can implement a privacy mode on the electronic device 820. Implementing a privacy mode can include disabling one or more privacy components 840 such as by severing the power source 823 from one or more privacy components 840. Severing the privacy components 840 from the power source 823 may prevent the privacy components 840 from receiving power which may cause the privacy components 840 to shut down. Advantageously, severing power to privacy components 840 may ensure that the privacy components 840 do not operate during a privacy mode. Advantageously, severing power to privacy components 840 may inhibit malicious software from operating the privacy components 840 during a privacy mode, for example, if malicious software were to attempt to override a privacy mode. Advantageously, severing power to privacy components 840 may inhibit access to data stored on the electronic device 820, such as from software applications executing on the electronic device 820 and/or from computing devices that are remote to the electronic device 820. For example, severing power to privacy components 840 may prevent data that originates and/or is stored on electronic device 820 from being communicated to remote devices. The controller 822 may be in communication with the switch 824A. The controller 822 can control an operation of the switch 824A. The controller 822 can cause the switch 824A to change states (e.g., transition between an open state and a closed state). The controller 822 can implement a privacy mode by causing the switch 824A to transition to an open state. The controller 822 can cause the switch 824A to transition to an open state in response to any of the conditions discussed herein prerequisite to implementing a privacy mode. The controller 822 can implement a privacy mode on the electronic device 820 by severing all of the privacy components 840 from the power source 823 (e.g., via switch 824A). In some implementations, the controller 822 can implement a privacy mode on the electronic device 820 by severing less than all of the privacy components 840 from the power source 823 (e.g., via switch 824A). For example, in some cases, the controller 822 may implement a privacy mode by severing power to only a few of the privacy components 840 such as to the WiFi component 841, the Bluetooth component 842, the cellular telephony component 848, the GNSS component 843, and/or the NFC component 844. Thus, the state of the actuator 821 and/or privacy mode state of the electronic device 820 can affect the GNSS component 843 and/or other privacy components 840.

Implementing a privacy mode can include disabling one or more privacy components 840 such as by holding one or more privacy components 840 in a reset mode. A privacy component 840 that is in a reset mode may fail to execute program instructions to perform operations. A privacy component 840 that is in a reset mode may ignore input data (e.g., electronic commands to perform operations). One or more of the privacy components 840 may comprise and/or be embodied as a chip configured to execute computer-executable instructions to perform operations. The chips of the privacy components 840 may be programmable. The chips may comprise an input pin corresponding to a reset mode. The chips may cause respective privacy components 840 to enter a reset mode based on input to their respective reset mode input pins. The controller 822 may be in communication with one or more privacy components 840, collectively or individually. For example, the controller 822 can communicate data to one or more of the privacy components 840. The controller 822 may be in communication with reset mode input pins on chips of respective privacy components 840. The controller 822 can communicate data to the privacy components 840 to drive reset mode input pins of respective chips to cause the privacy components 840 to enter a reset mode. For example, the controller 822 can drive the reset mode input pins high (or low depending on the implementation) to cause the privacy components 840 to enter a reset mode. The controller 822 can continuously drive the reset mode input pins during a privacy mode.

The controller 822 can implement a privacy mode on the electronic device 820 by causing all of the privacy components 840 to enter a reset mode. In some implementations, the controller 822 can implement a privacy mode on the electronic device 820 by causing less than all of the privacy components 840 to enter a privacy mode. For example, in some cases, the controller 822 may implement a privacy mode by causing only a few of the privacy components 840 such as the microphone 845, the altimeter 846, and/or the camera 847 to enter a reset mode. In some implementations, one or more of the privacy components 840 may be in communication with the power source 823 and may receive power from the power source 823 (via switch 824A) during a privacy mode, but may fail to execute program instructions to perform operations due to being in a reset mode.

The electronic device 820 can optionally include switch 824B. The switch 824B may include similar structural and/or operational features as switch 824A. The switch 824B may comprise one or more switches. The switch 824B may comprise an analog switch. The switch 824B may be embodied in circuitry. The switch 824B may comprise one or more MOSFETs. The switch 824B can transition between a plurality of states, such as an open state and a closed state. The switch 824A may be a single pole, double throw (SPDT) switch. The switch 824B can conduct an electrical signal (comprising data and/or energy) in a closed state. The switch 824A may inhibit the conduction of electrical signals in an open state. The switch 824B may be in communication with the device processor 825 and the privacy components 840, and may allow communication of data between the device processor 825 and the privacy components 840 when in a close state and may inhibit the communication of data in an open state.

Implementing a privacy mode can include disabling one or more privacy components 840 such as by severing the device processor 825 from one or more privacy components privacy components 840 (e.g., via switch 824B). Severing the privacy components 840 from the device processor 825 may prevent the privacy components 840 from sending to and/or receiving data from the device processor 825. For example, the device processor 825 may not be able to send commands to the privacy components 840 to control an operation of the privacy components 840 during a privacy mode and/or the privacy components 840 may not be able to send data (e.g., location data, data received from remote devices, etc.) during a privacy mode which can prevent data originating from the privacy components 840 from being stored in storage 827 and/or from being processed by the device processor 825. In some implementations, one or more of the privacy components 840 may be in communication with the power source 823 and may receive power from the power source 823 (via switch 824A) during a privacy mode, but may fail to communicate with the device processor 825. The controller 822 may be in communication with the switch 824B. The controller 822 can control an operation of the switch 824B. The controller 822 can cause the switch 824B to change states (e.g., transition between an open state and a closed state). The controller 822 can implement a privacy mode by causing the switch 824B to transition to an open state. The controller 822 can cause the switch 824B to transition to an open state in response to any of the conditions discussed herein prerequisite to initiating privacy mode. The controller 822 can implement a privacy mode on the electronic device 820 by severing all of the privacy components 840 from the device processor 825 (e.g., via switch 824B). In some implementations, the controller 822 can implement a privacy mode on the electronic device 820 by severing less than all of the privacy components 840 from the device processor 825 (e.g., via switch 824B).

Implementing a privacy mode can include disabling one or more privacy components 840 such as by interfering with a wireless communication signal or a wireless communication component, such as WiFi component 841, Bluetooth component 842, cellular telephony component 848, GNSS component 843, or NFC component 844. For example, the controller 822 (or the device processor 825) can interfere with a wireless communication antenna to decrease the antenna efficiency which can decrease the quality of a signal communicated from a wireless communication component or received at the wireless communication component.

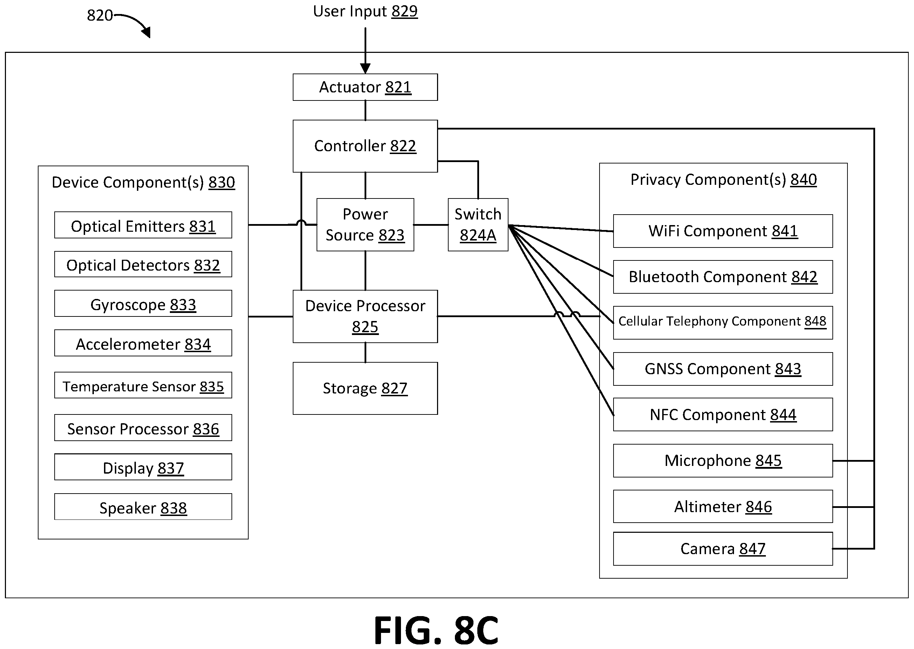

FIG. 8C is a block diagram illustrating an example implementation of an electronic device 820 which may include similar structural and/or operational features as shown and/or described with reference to FIG. 8B. In this example implementation, the switch 824A is in communication with the power source 823 as well as the WiFi component 841, the Bluetooth component 842, the cellular telephony component 848, the GNSS component 843, and the NFC component 844 (which may collectively be referred to as communication components). The controller 822 can cause the switch 824A to transition between states to disconnect the communication components from the power source 823 or to establish connection between the communication components and the power source 823. The controller 822 can implement a privacy mode (or a type of privacy mode) by causing the switch 824A to transition states to sever an electrical connection between the power source 823 and the communication components.

As shown in this example, the controller 822 is in communication with the microphone 845, the altimeter 846, and the camera 847. The controller 822 can communicate data to the microphone 845, the altimeter 846, and the camera 847, such as to cause the microphone 845, the altimeter 846, and the camera 847 to implement a reset mode. For example, the controller 822 can continuously drive an input pin (high or low depending on the implementation) on each of the microphone 845, the altimeter 846, and the camera 847 to cause the microphone 845, the altimeter 846, and the camera 847 to enter (and stay) in a reset mode. The controller 822 can implement a privacy mode (or a type of privacy mode) by causing the microphone 845, the altimeter 846, and/or the camera 847 to enter (and stay) in a reset mode.

As shown in this example implementation, the electronic device 820 can implement more than one type of privacy mode, and may implement a plurality of types of privacy mode simultaneously or independently. Examples are provided as follows. The electronic device 820 can implement a first type of privacy mode by disabling each of the privacy components 840. The electronic device 820 can implement a second type of privacy mode by disabling one or more of the WiFi component 841, the Bluetooth component 842, the cellular telephony component 848, the GNSS component 843, and the NFC component 844 (e.g., by severing their connection to power source 823) while not disabling one or more of the microphone 845, the altimeter 846, and the camera 847. The electronic device 820 can implement a third type of privacy mode by disabling one or more of the microphone 845, the altimeter 846, and the camera 847 (e.g., by holding them in reset mode) while not disabling one or more of the WiFi component 841, the Bluetooth component 842, the cellular telephony component 848, the GNSS component 843, and the NFC component 844.

The example implementation shown and/or discussed with reference to FIG. 8C is not intended to be limiting of the present disclosure. Any combination of the privacy components 840 may be selectively coupled to the power source 823 via the switch 824A and any combination of the privacy components 840 may be in communication with the controller 822 to receive data to force a reset mode. In some implementations, at least some of the privacy components 840 may be either coupled to the switch 824A or in communication with the controller 822, but not both. In some implementations, at least some of the privacy components 840 can be coupled to the switch 824A and can be in communication with the controller 822.

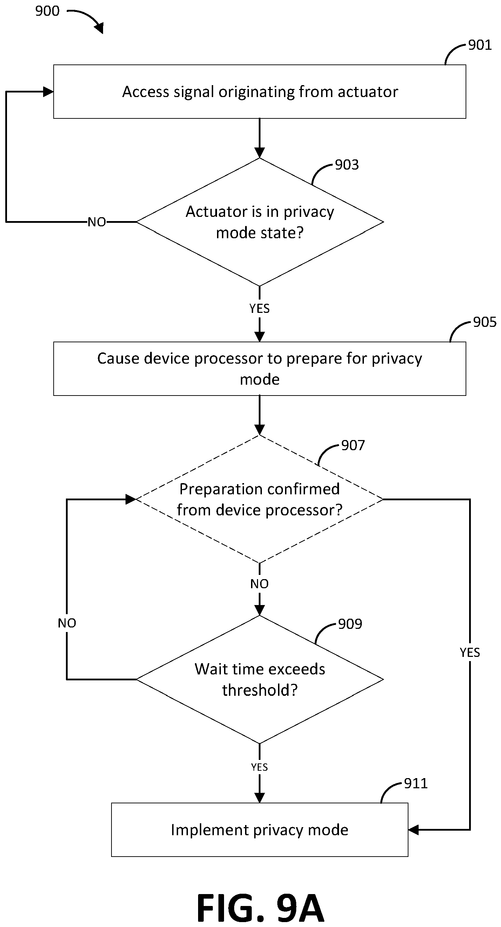

FIG. 9A is a flowchart illustrating an example process 900 of implementing a privacy mode on an electronic device. This process, in full or parts, can be executed by one or more hardware processors and/or controllers, whether they're associated with a singular or multiple computing devices, and even devices in remote or wireless communication. By way of example, the one or more hardware processors and/or controllers executing process 900 can be associated with any of the example electronic devices shown and/or described herein. For example, controller 822 shown and/or described herein can implement process 900 in full or in parts. The implementations of this process may vary and can involve modifications like omitting blocks, adding blocks, and/or rearranging the order of execution of the blocks. Process 900 serves as an example and isn't intended to restrict the present disclosure.

At block 901, an electronic device (e.g., one or more hardware processors and/or controllers of a computing device executing program instructions) can read an electronic state of an actuator which can correspond to a physical position of the actuator. The actuator can comprise any of the example actuators shown and/or described herein. The state of the actuator can change responsive to actuation of the actuator by a user.

At decision block 903 the electronic device can determine whether the actuator is in a privacy mode state. The actuator can exist in one or more states such as a privacy mode state and a non-privacy mode state. The electronic device can determine the state of the actuator from the actuator signal originating from and/or induced by the actuator at block 901. The electronic device can return to block 901 in response to determining that the actuator is not in a privacy mode state (which can coincide with a non-privacy mode state). The electronic device can proceed to block 905 in response to determining that the actuator is in a privacy mode state.

At block 905 the electronic device can cause a device processor to prepare for a privacy mode. For example, the controller can communicate a signal to the device processor to cause the device processor to prepare. The signal can be referred to as a notification signal. The device processor can be any of the example device processors shown and/or described herein. Implementing a privacy mode can include disabling components, severing power to components, resetting components, etc. which can cause processes that are executing to terminate. Terminating a process suddenly or without adequate preparation can cause loss of data, such as if the process is terminated asynchronously. The device processor can prepare the electronic device for privacy mode such as by terminating processes executing on the electronic device, which can include synchronously terminating the processes, storing data in storage, or the like. The device processor can terminate processes that are executing on the device processor or on other processors or circuits of the electronic device.

At decision block 907 the electronic device can optionally determine whether preparation for privacy mode has been confirmed as completed from the device processor. For example, upon completing preparation for privacy mode, the device processor can generate a signal (e.g., a confirmation signal) which can indicate that the electronic device is ready to safely enter privacy mode. Confirming that preparation for privacy mode is complete can include confirming that processes have been successfully terminated, that data has been properly stored, and/or that one or more components can be disabled (e.g., cut off from power or reset). If the electronic device determines at block 907, that the electronic device is prepared for a privacy mode (e.g., based on confirmation signal from the device processor), the electronic device can proceed to block 911. Block 907 can be optional at least because a controller of the electronic device that is executing block 907 may not be configured to receive communication from the device processor such as if the controller is isolated from the device processor or if the controller is in one-way communication with the device processor to send data to, but not receive data from, the device processor. If the electronic device does not determine at block 907, that the electronic device is prepared for a privacy mode (because a controller has not yet received a confirmation signal from a device processor and/or because the controller is not configured to receive data from the device processor), the electronic device can proceed to block 909.

At block 909 the electronic device can determine whether a wait time has exceeded a threshold. The electronic device can track a wait time (e.g., by setting a timer), to monitor a length of time spent preparing for privacy mode. For example, the electronic device can start a timer upon communicating a signal to the device processor to prepare for privacy mode at block 905. In some implementations, the electronic device can start a timer in response to determining that the actuator is in a privacy mode state at block 901. The threshold time can be between 1 second and 60 seconds, between 1 second and 30 seconds, between 1 second and 10 seconds, between 1 second and 5 seconds, between 1 second and 3 seconds, or between 1 second and 2 seconds. The threshold time can be between 0.1 seconds and 1 second or between 0.1 seconds and 0.5 seconds. The threshold time can be about 1 second, about 2 seconds, about 3 seconds, about 4 seconds, or about 5 seconds.

In response to determining that the wait time has not exceeded the threshold at block 909, the electronic device can return to block 907. Advantageously, allowing the electronic device a period of time to prepare to enter privacy mode reduce the likelihood of adverse effects, such as data loss, asynchronous process termination, applications crashing, etc.

In response to determining that the wait time has exceeded the threshold at block 909, the electronic device can proceed to block 911 to implement the privacy mode. Advantageously, implementing a privacy mode even if the electronic device is not prepared to safely enter the privacy mode can ensure privacy when desired. The electronic device can thus prioritize privacy over other concerns such as data loss, etc.

At block 911 the electronic device can implement a privacy mode. Implementing a privacy mode can include disabling one or more components of the electronic device, such as communication components (e.g., for near-field or far-field communication), location tracking components such as GNSS, microphones, altimeters, cameras, or the like. Disabling components during a privacy mode can include severing the components from a power source, holding the components in a reset mode, and/or severing the components from the device processor. In some implementations, certain components and/or processes of the electronic device can continue to operate. For example, physiological sensors can continue to generate sensor data during a privacy mode. As another example, the device processor of the electronic device can continue to operate during a privacy mode.

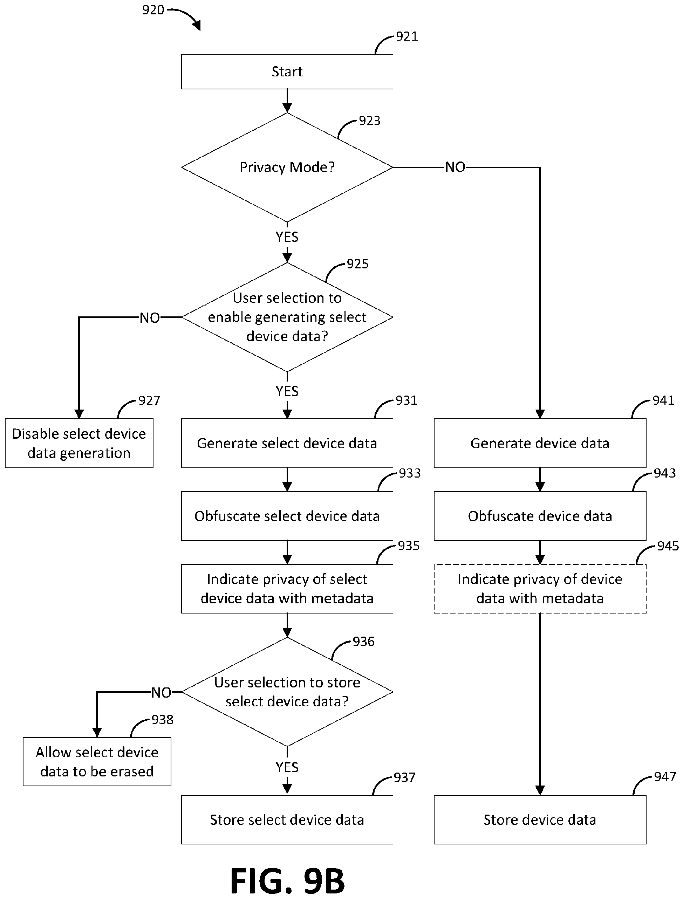

FIG. 9B is a flowchart illustrating an example process 920 of managing device data generated during privacy or non-privacy modes. This process, in full or parts, can be executed by one or more hardware processors, whether they're associated with a singular or multiple computing devices, and even devices in remote or wireless communication. By way of example, the one or more hardware processors executing process 920 can be associated with any of the example electronic devices shown and/or described herein. The implementations of this process can vary and can involve modifications like omitting blocks, adding blocks, and/or rearranging the order of execution of the blocks. Process 920 serves as an example and isn't intended to restrict the present disclosure. Process 920, or portions thereof, can be implemented during a privacy mode and/or during a non-privacy mode.

Process 920 can commence at block 921. At block 923, an electronic device (e.g., one or more hardware processors of a computing device executing program instructions) can determine whether the electronic device is in a privacy mode, such as any of the example privacy modes described herein. The electronic device can determine the occurrence of a privacy mode based on at least the state of an actuator, such as a mechanically actuated actuator such as a switch that can generate signals (e.g., change voltage) responsive to actuation and indicative of the actuator state. For example, the electronic device can read the state of the actuator to determine whether the actuator is actuated. The process 920 can proceed to block 925 responsive to determining the electronic device is in a privacy mode. The process 920 can proceed to block 941 responsive to determining the electronic device is in a non-privacy mode.

At decision block 925 the electronic device can determine whether a user has selected to enable generating select device data (e.g., during the privacy mode). For example, a user can select for the electronic device to continue generating certain data during a privacy mode while not generating other data during the privacy mode. Select device data can include sensor data originating from one or more sensors such as any of the example sensors shown and/or described herein such as physiological data originating from physiological sensors. Select device data can include processed and/or unprocessed sensor data. Select device data can include physiological parameters derived from physiological data. Select device data can include audio data originating from microphones, image data originating from cameras, altitude data originating from altimeters, location data originating from a GNSS component, etc.

In some implementations, a user can select to enable or disable generating select device data with a hardware actuator disposed on the electronic device, such as a button or switch. In some implementations, a user can select to enable or disable generating select device data via a graphical user interface such as can be displayed on a screen of the electronic device. In some implementations, a user can toggle between enabling and disabling generating select device data as desired. If the electronic device determines at decision block 925 that the user has not selected to enable generating select device data or has selected to disable generating select device data, the process can proceed to block 927. If the electronic device determines at decision block 925 that the user has selected to enable generating select device data, the process can proceed to block 931.

At block 927 the electronic device can disable generation of select device data. For example, the electronic device can disable one or more sensors from generating select device data. The electronic device can disable the sensors from generating data according to any of the example implementations described herein, such as severing power to the sensors or holding the sensors in a reset mode. In some implementations, the electronic device can disable select data generation by not providing instructions to the sensors to generate the data (while allowing the sensors to receive power and remain operational). In some implementations, the electronic device can disable select data generation by severing electrical communication between the sensors and a processor (while allowing the sensors to continue to operate).

At block 931 the electronic device can generate select device data during the privacy mode. For example, one or more sensors can continue to generate (or not generate) data according to normal operational functionality, such as generating data intermittently at a certain frequency, generating data based on time conditions, generating data based on location conditions, generating data in response to user requests (e.g., spot checks), generating data automatically, generating data based on health conditions (e.g., determined from real-time physiological sensor data), and the like. Advantageously, the electronic device can continue to generate select device data, such as physiological data, during a privacy mode to preserve data continuity. Such select data that is generated during a privacy mode can still remain secure during the privacy mode at least because other operations, such as communication from the electronic device are disabled during the privacy mode, and can also remain secure after the privacy mode as will be described in greater detail throughout process 920 and process 950.

From block 931, the process 920 can proceed to block 933 and other blocks. In some implementations, certain aspects of process 920, including block 933 or other operations appearing in FIG. 9B after block 933, such as blocks 935, 936, 937, or 938, can occur during the privacy mode discuss at block 923 or after the privacy mode has terminated, such as during a non-privacy mode. Moreover, the order of blocks 933 and 935 can be reversed from what is shown in FIG. 9B and/or one or more of blocks 933 and 935 can occur after decision block 936 and/or after block 937.

At block 933, the electronic device can obfuscate the select device data generated during the privacy mode. For example, the electronic device can encrypt the device data according to one or more encryption protocols with one or more encryption algorithms. The electronic device can encrypt the device data with an encryption key which can be stored locally on the electronic device. Advantageously, encrypting the device data can prevent the device data from being decrypted, deciphered, or useable by another computing device, for example, if the device data were communicated to another computing device. Advantageously, encrypting the device data can prevent unauthorized program applications executing on the electronic device (e.g., malware) from decrypting or using the device data.

At block 935, the electronic device can indicate a privacy status of the select device data with metadata. For example, the electronic device can create a tag, marker, or identifier to assign to the device data to indicate that the device data is private. Data can be private if it was generated during a privacy mode. Data can be non-private if it was generated during a non-privacy mode. In some implementations, metadata can indicate various levels of privacy status (e.g., not private, private level 1, private level 2), such as may be based on a type of privacy mode during which the data was generated. In some implementations, the electronic device can update existing metadata or delete existing metadata at block 935 to accurately reflect the privacy status of the data. Advantageously, the electronic device may be able to quickly identify whether device data is private, based on its metadata, and can manage the device data accordingly.

At decision block 936 the electronic device can determine whether a user has selected to store the select device data generated during the privacy. The user selection to store the device data can include storing the device data in long-term storage. In some implementations, a user can select to store the select device data with a hardware actuator disposed on the electronic device, such as a button or switch. In some implementations, a user can select to store the select device data via a graphical user interface such as can be displayed on a screen of the electronic device. In some implementations, a user can toggle between storing and not storing the select device data as desired. If the electronic device determines at decision block 925 that the user has selected to store the select device data, the process 920 can proceed to block 937. If the electronic device determines at decision block 926 that the user has not selected to store the select device data, the process 920 can proceed to block 938.

At block 938 the electronic device can allow select device data to be erased and/or may not store the select device data. For example, the electronic device can store the device data as non-persistent data in short term storage and can allow the device data to be erased from short-term storage without storing the data as persistent data in long-term storage. Short-term storage can include volatile memory such as registers, caches, buffers, and/or RAM. The electronic device can periodically erase data from short-term storage, such as based on clock timing.

At block 937 the electronic device can store the select device data, for example, as persistent data in long-term storage. Long-term storage can include non-volatile storage such as hard drives. Persistent data (e.g., stored in long-term storage) can persist after powering off the electronic device and/or terminating a process or application having the data executing on the electronic device. The long-term storage can be local to the electronic device. In some implementations, the electronic device can store the device data as organized data structures based on metadata. Data structures can include tables, rows, and columns. For example, the electronic device can sort the device data within a table based on metadata and/or can separate the device data into various tables or columns based on metadata. Advantageously, storing the device data within formatted data structures based on its metadata can increase electronic processing efficiencies such as faster data queries, data retrieval, and/or data analysis. In some implementations, the electronic device can store the device data in various locations in storage based on whether it is private or non-private according to its metadata such that the private data may be separate from non-private data. Storing data at various locations can include storing data at different addresses in memory, which may or may not be within a same partition. For example, private data can be stored at location in storage corresponding to a first memory address and the non-private data can be stored at another location in storage corresponding to another memory address. In some implementations, the electronic device can store private data and non-private data (e.g., depending on its metadata) within a same partition in storage. Advantageously, the electronic device may not need to divide the storage into separate partitions to store the device data based on whether it is private at least because the electronic device may be able to track private data and non-private data by its metadata rather than having to track based on a partition in which it is stored. In some implementations, the electronic device can store the device data at various locations within separate partitions based on whether the data is private or non-private. In some implementations, the electronic device can store the device data within separate storage devices based on whether the data is private or non-private.

From block 923, the process 920 can proceed to block 941 responsive to determining the electronic device is in a non-privacy mode. Thus, blocks 941, 943, 945, and 947 can execute during a non-privacy mode.

At block 941 the electronic device can generate device data. For example, one or more physiological sensors, cameras, microphones, altimeters, and/or location devices can continue to generate (or not generate) data according to normal operational functionality, such as generating data intermittently at a certain frequency, generating data based on time conditions, generating data based on location conditions, generating data in response to user requests (e.g., spot checks), generating data automatically, generating data based on health conditions (e.g., determined from real-time physiological sensor data), and the like. Device data generated at block 941 can include physiological data originating from physiological sensors, audio data originating from microphones, image data originating from cameras, altitude data originating from altimeters, location data originating from a GNSS component, etc.

At block 943, the electronic device can obfuscate the device data generated during the non-privacy mode. For example, the electronic device can encrypt the device data according to one or more encryption protocols with one or more encryption algorithms. The electronic device can encrypt the device data with an encryption key which can be stored locally on the electronic device. Advantageously, encrypting the device data can prevent the device data from being decrypted, deciphered, or usable by another computing device, for example, if the device data were communicated to another computing device. Advantageously, encrypting the device data can prevent unauthorized program applications executing on the electronic device (e.g., malware) from decrypting or using the device data.

At block 945, the electronic device can optionally indicate a privacy status of the device data with metadata. For example, the electronic device can create a tag, marker, or identifier to assign to the device data to indicate that the device data is private. Data may be private if it was generated during a privacy mode. Data may be non-private if it was generated during a non-privacy mode. In some implementations, all health-related data can be treated as confidential according to government health data restrictions, however such confidential health-related data generated during a privacy mode can be further indicated as being private to distinguish other confidential health-related data generated during a non-privacy mode. In some implementations, the electronic device can update existing metadata or delete existing metadata at block 945 to accurately reflect the privacy status of the data. Advantageously, the electronic device may be able to quickly identify whether device data is private or non-private, based on its metadata, and can manage the device data accordingly. In addition or alternatively to using metadata, data indicated as private can be encrypted using a different encryption key than data not indicated as private.

At block 947 the electronic device can store the select device data, for example, as persistent data in long-term storage as discussed in greater detail at block 937.

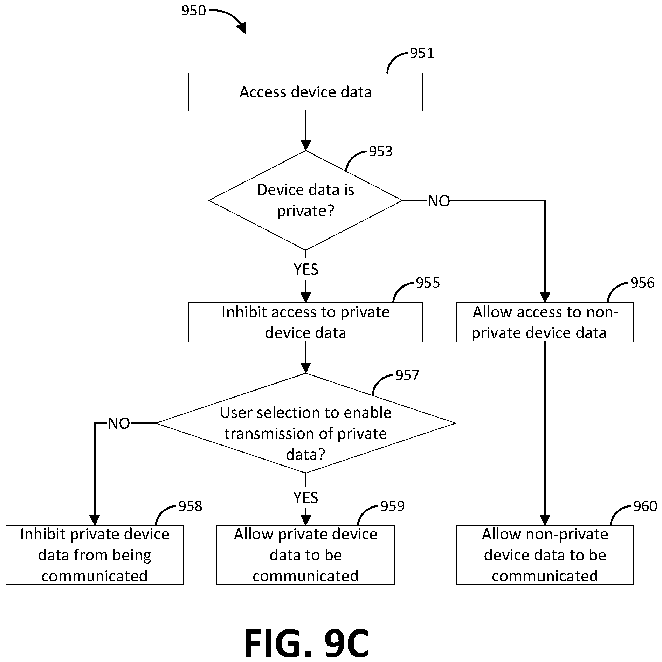

FIG. 9C is a flowchart illustrating an example process 950 of managing access to and communication of device data. This process, in full or parts, can be executed by one or more hardware processors, whether they're associated with a singular or multiple computing devices, and even devices in remote or wireless communication. By way of example, the one or more hardware processors executing process 950 can be associated with any of the example electronic devices shown and/or described herein. The implementations of this process can vary and can involve modifications like omitting blocks, adding blocks, and/or rearranging the order of execution of the blocks. Process 950 serves as an example and isn't intended to restrict the present disclosure.

Process 950, or portions thereof, can be implemented during a privacy mode and/or during a non-privacy mode. In some implementations, process 950, or portions thereof, can be implemented during a non-privacy mode such as when a communication component of an electronic device is powered on and/or operating.

At block 951, an electronic device (e.g., one or more hardware processors of a computing device executing program instructions) can access device data. Accessing device data can include retrieving the device data from storage (e.g., local storage on the electronic device). Accessing device data can include receiving the device data (e.g., at a processor) from one or more sensors such as in real-time as the device data is generated at the sensors. Device data can include sensor data originating from one or more sensors such as any of the example sensors shown and/or described herein such as physiological sensors, microphones, altimeters, cameras, etc. Device data can include data originating from devices or components of the electronic device, such as location data originating from a GNSS component. Device data can include physiological data, audio data, and/or image data. Device data can include processed or unprocessed data. For example, device data can include raw and/or unprocessed sensor data and could also include processed sensor data such as physiological parameters derived from physiological data. Device data can originate from sensors local to the electronic device. Device data can originate from computing devices remote to the electronic device. Device data can include historical data previously generated and/or real-time data generated at substantially the same time as execution of process 950 or portions thereof.

At decision block 953, the electronic device can determine whether the device data is private. The electronic device can determine whether the device data is private, based on at least, metadata of the device data. For example, metadata of the device data can include an identifier that indicates whether the device data is private. Device data can be private if it was generated, processed, stored, and/or received during a privacy mode. For example, private device data can include sensor data that was generated by sensors on the electronic device while the electronic device was in a privacy mode. Advantageously, the electronic device can be able to quickly determine whether the device data is private based on the metadata which can result in faster processing and/or improved processing efficiency. In some implementations, the electronic device can additionally or alternatively, at block 953, determine whether the device data is not private. In some implementations, device data is either private or not private. In some implementations, device data cannot be both private and not private. If the electronic device determines that the device data is private, the electronic device can proceed to block 955. If the electronic device determines that the device data is not private, the electronic device can proceed to block 956.

At block 955, the electronic device can inhibit access to the private device data. Inhibiting access to the device data can include preventing remote computing devices from accessing the device data. Inhibiting access to the device data can include preventing program applications executing on the electronic device from accessing the device data. Program applications can include software and/or firmware applications executing program instructions on the electronic device to cause the electronic device to perform various operations. Program applications can be installed on the electronic device such as downloaded from a remote server. Program applications can include third-party applications executing on the electronic device. Program applications can include mobile apps. In some implementations, the electronic device can prevent program applications from accessing the device data regardless of any permission granted to the program applications by the user to access data, such as user authorizations during initial installation of the software app on the electronic device. In some implementations, the electronic device can prevent access to the device data to all program applications currently on the electronic device and/or to all program applications that can be installed on the electronic device in the future. In some implementations, the electronic device can inhibit access to the device data to less than all program applications on the electronic device. The electronic device can inhibit access to the device data based on the metadata of the device data or separate encryption key. The electronic device can determine which program application can access which device data based on the metadata or level/type of encryption. For example, device data metadata or level/type of encryption can indicate which program applications can access the device data and which program applications cannot access the device data.

At block 956, the electronic device can allow access to non-private device data. Allowing access to the device data can include allowing remote computing devices to access the device data. Allowing access to the device data can include allowing software applications, such as third-party applications, executing on the electronic device to access the device data. In some implementations, the electronic device can allow access to the device data to all software applications currently on the electronic device and/or to all software applications that can be installed on the electronic device in the future. In some implementations, the electronic device can allow access to the device data to less than all software applications currently on the electronic device, such as determined by the device data metadata.

At decision block 957, the electronic device can determine whether a user has selected to enable private data to be communicated from the electronic device. In some implementations, a user can select to enable or disable private data transmission with a hardware actuator disposed on the electronic device, such as a button or switch. In some implementations, a user can select to enable or disable private data transmission via a graphical user interface such as can be displayed on a screen of the electronic device. In some implementations, a user can toggle between enabling and disabling private data transmission as desired. If the electronic device determines at decision block 957 that the user has not selected to enable private data transmission or has selected to disable private data transmission, the process can proceed to block 958. If the electronic device determines at decision block 957 that the user has selected to enable private data transmission, the process can proceed to block 959.

At block 958, the electronic device can inhibit the private device data from being communicated to remote computing devices. For example, the electronic device can cause a communication component of the electronic device to not communicate the device data.

At block 959, the electronic device can allow or cause the private device data to be communicated to remote computing devices. For example, the electronic device can cause a communication component of the electronic device to communicate the device data. Communicating the device data can include transmitting the device data (for example, wirelessly) to one or more remote computing devices or servers. The electronic device can communicate the private device data over a secure channel. Communicating data over a secure channel can inhibit eavesdropping (e.g., to preserve confidentiality) and/or can inhibit data tampering (e.g., to preserve authenticity). Communicating data over a secure channel can include relying on a trusted third party. For example, the electronic device can communicate data to a trusted third party device which can in turn communicate the data to a destination computing device. A trusted third party can be a certificate authority (CA) that issues certificates indicating its security. The electronic device can communicate private data to a trusted third party responsive to receiving a security certificate from the trusted third party indicating the third party can be trusted to manage data communication securely. Communicating data over a secure channel can include the communicating parties relying on trust between them. For example, the electronic device can communicate data to a receiving computing device with a key exchange protocol, such as a D-H key exchange protocol. For example, the electronic device and the receiving computing device can exchange information between them that is useable and/or combinable to generate a key that is only known to them that can be used to communicate data between them. The electronic device can encrypt the private device data prior to communicating the device data, even when communicating the data over a secure channel. Thus, the electronic device can communicate encrypted data over a secure channel.

Device metadata can persist across devices. For example, device data metadata can continue to exist on a receiving computing device after the device data is communicated from the electronic device to the receiving computing device. A receiving computing device can determine the privacy status of device data based on its metadata and can manage the data appropriately, including storing the data in a certain location based on its privacy status, displaying the data with an indication to indicate it is private, etc.

At block 960, the electronic device can allow or cause the non-private device data to be communicated to remote computing devices. For example, the electronic device can cause a communication component of the electronic device to communicate the device data. Communicating the device data can include transmitting the device data (for example, wirelessly) to one or more remote computing devices or servers. The electronic device can communicate non-private device data over a secure channel or in some implementations can communicate non-private device data over an insecure channel which can be subject to eavesdropping and/or tampering. Communicating data over an insecure channel can improve communication speed, reduce processing requirements, and/or improve computational efficiency. In some implementations, the electronic device can communicate non-private device data over an authentic channel (which can be resistant to tampering but which can be subject to eavesdropping). In some implementations, the electronic device can communicate non-private device data over a confidential channel (which can be resistant to eavesdropping but which can be subject to tampering). The electronic device can encrypt non-private device data prior to communicating said data. Thus, the electronic device can securely communicate the encrypted non-private device data over an insecure channel.

Process 950 is provided as an example and is not intended to be limiting of the present disclosure. In some implementations, the processor can communicate and/or not communicate device data regardless of whether the device data is private.

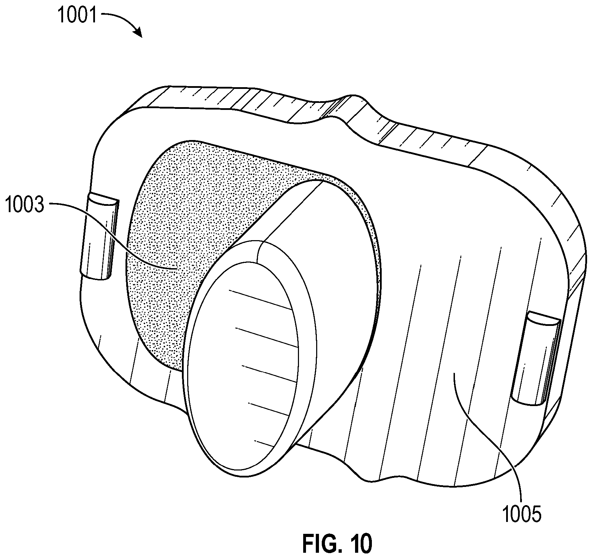

FIG. 10 is a perspective view illustrating an example implementation of an actuator 1001. The actuator 1001 can include similar structural and/or operational features as any of the other example actuators shown and/or discussed herein. The actuator 1001 can be implemented in an electronic device such as any of the example electronic devices shown and/or discussed herein. The actuator 1001 can include a first portion 1003 and a second portion 1005. The first portion 1003 can include a first coloring or shading such as red, orange, yellow, green, blue, purple, black, gray, white, brown, or any other color or shade. The second portion can include a second coloring or shading such as red, orange, yellow, green, blue, purple, black, gray, white, brown, or any other color or shade. In some implementations, the first portion 1003 can include a same coloring or shading as the second portion. In some implementations, the first portion 1003 can include a different coloring or shading than the second portion.

The first portion 1003 can be visible when the actuator 1001 is in a certain state and may not be visible when the actuator 1001 is in another state. For example, the first portion 1003 can be exposed to an exterior surface when in one state and not exposed to an exterior surface when in another state. The second portion 1005 can be visible when the actuator 1001 is in a certain state and may not be visible when the actuator 1001 is in another state. The first portion 1003 may not be visible when the second portion 1005 is visible and vice versa.

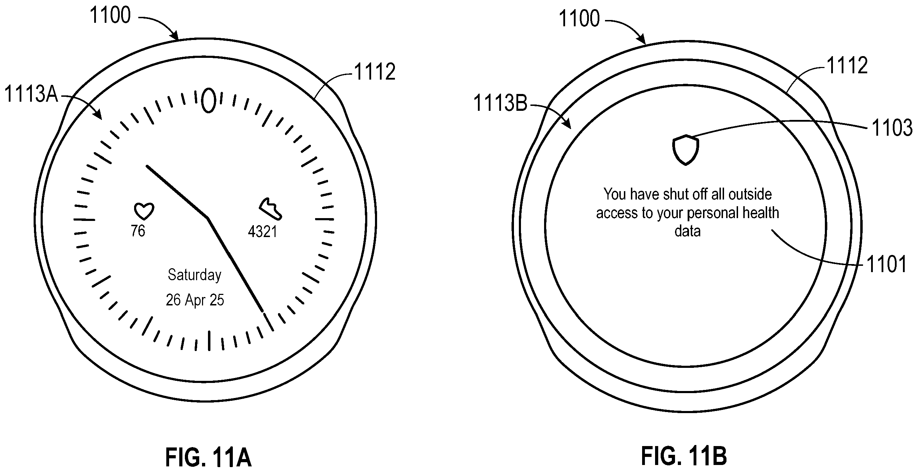

FIGS. 11A-11D illustrate an electronic device 1100 with display screen 1112 showing example user interfaces 1113A-1113D. The display screen 1112 can be a digital displays such as LED and/or LCD displays. A hardware processor, such as any of the example hardware processors shown and/or described herein, can generate user interface data for rendering the user interfaces 1113A-1113D shown and/or described in FIGS. 11A-11D. In some implementations, a dedicated controller (e.g., controller 822 shown and/or described herein) can cause a hardware processor to generate user interface data for rendering any portions of user interfaces 1113A-1113D.

As shown in FIG. 11A, the user interface 1113A can display time, such as an analog watch face, date, and indicia of physiological data such as icons and physiological parameters such as heart rate, step counter, pulse rate, respiration rate, SpO2, etc. The user interface 1113A can be displayed during a non-privacy mode.

As shown in FIG. 11B, the user interface 1113B can include a first indication 1101 that the electronic device has entered a privacy mode. The first indication 1101 can indicate to a user what will happen to certain data during the privacy mode. The user interface 1113B can include a second indication 1103 to indicate that the electronic device is in a privacy mode. The second indication 1103 can be a symbol. The first indication 1101 and/or second indication 1103 can be displayed responsive to first entering a privacy mode, such as for the first 10 seconds of privacy mode, for the first minute of privacy mode, etc. In some implementations, a dedicated controller (e.g., controller 822 shown and/or described herein) can cause electronic device 1100 to display first indication 1101 and/or second indication 1103 such as by instructing a hardware processor to generate user interface data for rendering first indication 1101 and/or second indication 1103. Controlling the display of first indication 1101 and/or second indication 1103 with a dedicated controller can advantageously prevent electronic device 1100 from erroneously displaying first indication 1101 and/or second indication 1103 (e.g., when not in a privacy mode) such as if the electronic device 1100 is hacked. As discussed, a dedicated controller can be impervious to hacking at least because a dedicated controller may be non-programmable. A hardware processor, such as device processor 825 shown and/or described herein, may not control whether certain portions of user interfaces are displayed (and a dedicated controller may have sole control over displaying those portions) although, in some cases, the device processor may be involved in generating user interface data for rendering those portions responsive to instructions to do so. In some implementations, the dedicated controller can generate user interface data for rendering portions of user interfaces.

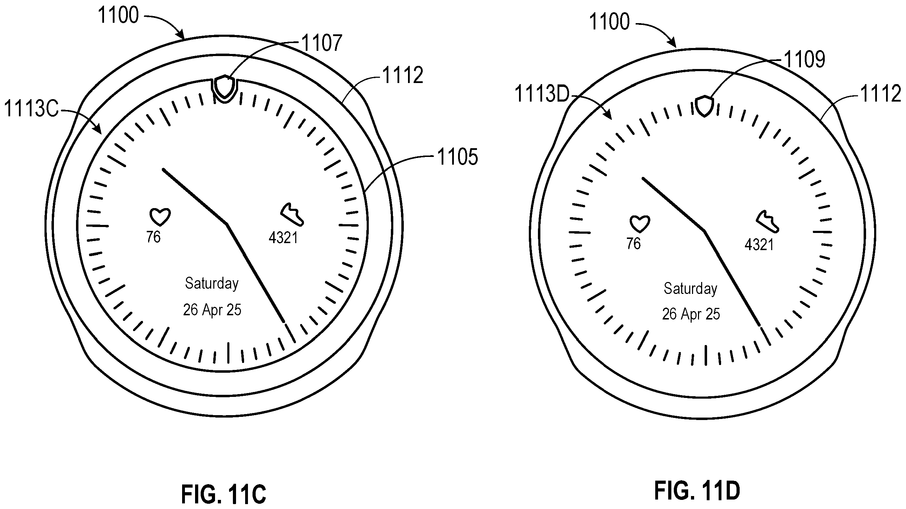

As shown in FIG. 11C, the user interface 1112C can include time, date, and indicia of physiological data such as icons and/or physiological parameters such as heart rate and step counter. The user interface 1112C can be displayed during a privacy mode. The indicia of physiological data can correspond to real-time data originating from physiological sensors on the electronic device 1100 during the privacy mode. Advantageously, a user can continue to view and monitor their physiological status during a privacy mode. The user interface 1112C can include a first indication 1105 that the electronic device is in a privacy mode. The first indication 1105 can be annular or semi-annular. In some implementations, the first indication 1105 can be rectangular. The first indication 1105 can surround user interface 1112C around a perimeter of user interface 1112C. The first indication 1105 can circumscribe display and/or watch face. In some implementations, the display screen 1112 can display the first indication 1105 for an entire duration of a privacy mode. In some implementations, the display screen 1112 can display the first indication 1105 only upon activation of the privacy mode. For example, the display screen 1112 can display the first indication 1105, for a length of time after entering privacy mode and may not display the first indication 1105 for an entire duration of the privacy mode. The user interface 1112C can include a second indication 1107 that the electronic device is in a privacy mode. The display screen 1112 can display the second indication 1107 for an entire duration of the privacy mode. In some implementations, a dedicated controller (e.g., controller 822 shown and/or described herein) can cause electronic device 1100 to display indications 1105 and/or 1107 such as by instructing a hardware processor to generate user interface data for rendering indications 1105 and/or 1107. Controlling the display of indications 1105 and/or 1107 with a dedicated controller can advantageously prevent electronic device 1100 from erroneously displaying indications 1105 and/or 1107 (e.g., when not in a privacy mode) such as if the electronic device 1100 is hacked.

As shown in FIG. 11D, the display screen 1112 can display time, date, and indicia of physiological data such as icons and/or physiological parameters such as heart rate and step counter which can correspond to real-time physiological data and/or historical physiological data. The user interface 1113D can include an indication 1109. In some implementations, the indication 1109 can indicate that the electronic device is currently in a privacy mode. The display screen 1112 can display the indication 1109 for an entire duration of the privacy mode. In some implementations, the indication 1109 can indicate that indicia of physiological data displayed on the display screen 1112 (e.g., heart rate, step counter) corresponds to physiological data that was generated during a privacy mode (e.g., is private data). Electronic device 1100 can cause display screen 1112 to display indication 1109 only when private data is displayed. In some implementations, the indicia of physiological data displayed on the display screen 1112D (e.g., heart rate, step counter) corresponds to physiological data originating from another electronic device and received at the electronic device 1100 (e.g., via wireless communication). The electronic device 1100 can determine that physiological data originating from another electronic device is private (and can thus display the indication 1109) based on metadata of the physiological data, as discussed herein. In some implementations, a dedicated controller (e.g., controller 822 shown and/or described herein) can cause electronic device 1100 to display indication 1109 such as by instructing a hardware processor to generate user interface data for rendering indication 1109. Controlling the display of indication 1109 with a dedicated controller can advantageously prevent electronic device 1100 from erroneously displaying indication 1109 (e.g., when displayed data is not private data) such as if the electronic device 1100 is hacked.

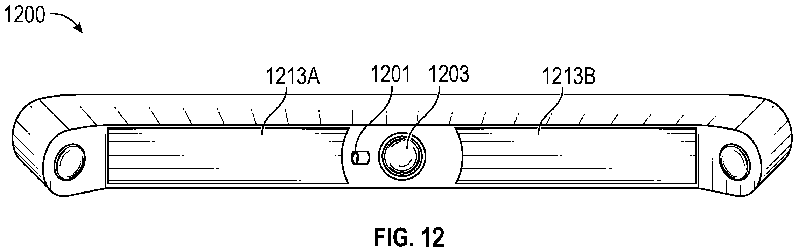

FIG. 12 illustrates an example electronic device 1200 implemented as a soundbar. The electronic device 1200 can include one or more speakers 1213 (e.g., speaker 1213A, speaker 1213B), a camera 1203, and an actuator 1201 (which may also be referred to as a privacy switch). The speakers 1213 can emit audio. The camera 1203 can collect images. The electronic device 1200 can communicate with separate devices via a wireless communication protocol. For example, the electronic device 1200 can communicate images captured by the camera 1203 to separate computing devices. As another example, the electronic device 1200 can cause the speakers 1213 to emit audio that is received from separate computing devices. The actuator 1201 can control one or more operations of the electronic device 1200 to secure a user's data. For example, actuation of the actuator 1201 can enable/disable the camera 1203 to inhibit the camera 1203 from capturing images. As another example, the actuation of the actuator 1201 can allow/inhibit the electronic device 1200 from communicating with separate devices to send and/or receive data from separate devices. In some aspects, actuation of the actuator 1201 may not affect operation of some components of the electronic device 1200 such as the speakers 1213.

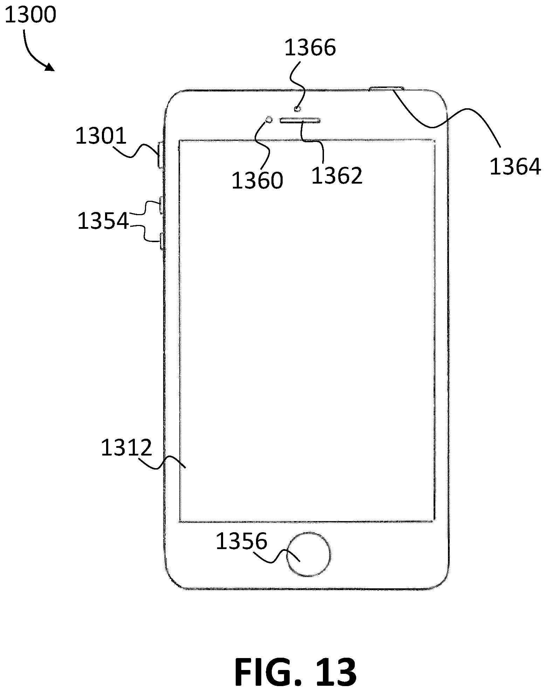

FIG. 13 illustrates a perspective view of an example electronic device 1300 implemented as a mobile phone. In this example, the electronic device 1300 includes a display 1312 which can be a touch screen, an audio control 1354, a control button 1356, a microphone 1366, a camera 1360, a pairing button 1364 for establishing wireless connectivity with separate devices, and a speaker 1362. The electronic device 1300 can also include an actuator 1301 that can facilitate the protection of a user's data. The actuator 1301 can be a slideable switch or a button. The electronic device 1300 can include fewer, additional, or different components. The electronic device 1300 can communicate with other separate devices via a wireless communication protocol. Actuation of the actuator 1301 can enable/inhibit the electronic device 1300 from communicating with separate computing devices. In some implementations, actuation of the actuator 1301 can disable one or more components of the electronic device such as the camera 1360 and/or the microphone 1366. In some cases, actuation of the actuator 1301 may not affect operation of some components of the electronic device 1300 such as the display 1312 and/or the speaker 1362. In some implementations, actuation of the actuator 1301 can disable the electronic device 1300 from communicating with separate devices.

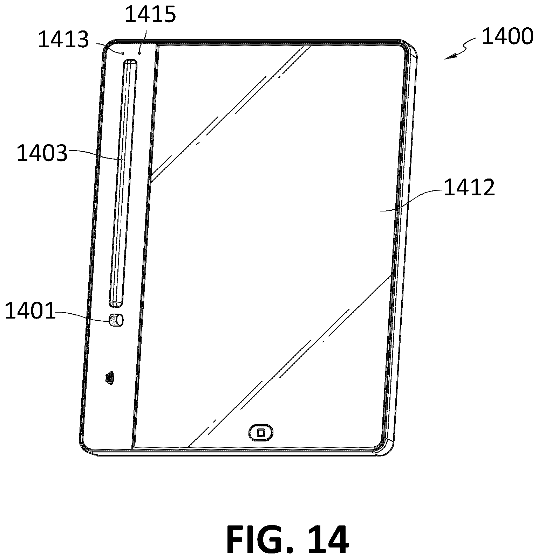

FIG. 14 illustrates an example electronic device 1400 implemented as a monitoring hub (which may also be referred to as a tablet, a health monitoring device, a display device, etc.). Electronic device 1400 can include a display 1412, a status indicator 1403, a microphone 1413, a camera 1415, and an actuator 1401. Display 1412 can display indicia of physiological data such as charts, trend lines, graphs, physiological parameters, gauges, dials, alerts, notifications, medication protocol, or other health related information. Microphone 1413 can detect audio. Camera 1415 can collect images. Status indicator 1403 can indicate a status of the electronic device 1400 including whether the electronic device 1400 is in a privacy mode or a non-privacy and/or what type of privacy mode the electronic device 1400 is in. Actuator 1401 can enable/disable a privacy mode of the electronic device 1400. For example, actuation of the actuator 1401 can disable one or more components of the electronic device 1400 such as the microphone 1413 and/or the camera 1415. As another example, actuation of the actuator 1401 can disable communication of the electronic device 1400 with separate computing devices. In some implementations, actuation of the actuator 1401 may not affect operation of at least some of the components of the electronic device such as the display 1412 and/or the status indicator 1403.

### Additional Considerations

Certain categories of persons, such as caregivers, clinicians, doctors, nurses, and friends and family of a user, may be used interchangeably to describe a person providing care to the user. Furthermore, patients or users used herein interchangeably refer to a person who is wearing a sensor or is connected to a sensor or whose measurements are used to determine a physiological parameter or a condition. Parameters may be, be associated with, and/or be represented by, measured values, display icons, alphanumeric characters, graphs, gages, power bars, trends, or combinations. Real time data may correspond to active monitoring of a user, however, such real time data may not be synchronous to an actual physiological state at a particular moment. Measurement value(s) of a parameter and the parameter used herein such as, SpO2, RR, PaO2 and the like, unless specifically stated otherwise, or otherwise understood with the context as used is generally intended to convey a measurement or determination that is responsive to the physiological parameter.

Although certain implementations and examples have been described herein, it will be understood by those skilled in the art that many aspects of the systems and devices shown and described in the present disclosure may be differently combined and/or modified to form still further implementations or acceptable examples. All such modifications and variations are intended to be included herein within the scope of this disclosure. A wide variety of designs and approaches are possible. No feature, structure, or step disclosed herein is essential or indispensable. The various features and processes described herein may be used independently of one another, or may be combined in various ways. For example, elements may be added to, removed from, or rearranged compared to the disclosed example implementations. All possible combinations and sub-combinations are intended to fall within the scope of this disclosure.

Any methods and processes described herein are not limited to any particular sequence, and the blocks or states relating thereto can be performed in other sequences that are appropriate. For example, described blocks or states may be performed in an order other than that specifically disclosed, or multiple blocks or states may be combined in a single block or state, or certain method or process blocks may be omitted, or certain blocks or states may be performed in a reverse order from what is shown and/or described. The example blocks or states may be performed in serial, in parallel, or in some other manner. Blocks or states may be added to or removed from the disclosed example implementations.

The methods disclosed herein may include certain actions taken by a practitioner; however, they can also include any third-party instruction of those actions, either expressly or by implication.

The methods and tasks described herein may be performed and fully automated by a computer system. The computer system may, in some cases, include multiple distinct computers or computing devices (e.g., physical servers, workstations, storage arrays, cloud computing resources, etc.) that communicate and interoperate over a network to perform the described functions. The computer system may, in some cases, include a single computing device, processor, controller, or the like. Each such computing device typically includes a processor (or multiple processors) that executes program instructions or modules stored in a memory or other non-transitory computer-readable storage medium or device (e.g., solid state storage devices, disk drives, etc.). The various functions disclosed herein may be embodied in such program instructions, and/or may be implemented in application-specific circuitry (e.g., ASICs or FPGAs) of the computer system. Where the computer system includes multiple computing devices, these devices may, but need not, be co-located. The results of the disclosed methods and tasks may be persistently stored by transforming physical storage devices, such as solid state memory chips and/or magnetic disks, into a different state. The computer system may be a cloud-based computing system whose processing resources are shared by multiple distinct entities or other users. The systems and modules may also be transmitted as generated data signals (for example, as part of a carrier wave or other analog or digital propagated signal) on a variety of computer-readable transmission mediums, including wireless-based and wired/cable-based mediums, and may take a variety of forms (for example, as part of a single or multiplexed analog signal, or as multiple discrete digital packets or frames).

Many other variations than those described herein will be apparent from this disclosure. For example, depending on the implementation, certain acts, events, or functions of any of the algorithms described herein can be performed in a different sequence, can be added, merged, or left out altogether (for example, not all described acts or events are necessary for the practice of the algorithms). Moreover, in certain implementations, acts or events can be performed concurrently, for example, through multi-threaded processing, interrupt processing, or multiple processors or processor cores or on other parallel architectures, rather than sequentially. In addition, different tasks or processes can be performed by different machines and/or computing systems that can function together.

Various illustrative logical blocks, modules, routines, and algorithm steps that may be described in connection with the disclosure herein can be implemented as electronic hardware (e.g., ASICs or FPGA devices), computer software that runs on computer hardware, or combinations of both. Various illustrative components, blocks, and steps may be described herein generally in terms of their functionality. Whether such functionality is implemented as specialized hardware versus software running on general-purpose hardware depends upon the particular application and design constraints imposed on the overall system. The described functionality can be implemented in varying ways for each particular application, but such implementation decisions should not be interpreted as causing a departure from the scope of the disclosure.

Moreover, various illustrative logical blocks and modules that may be described in connection with the implementations disclosed herein can be implemented or performed by a machine, such as a general purpose processor, a digital signal processor ("DSP"), an application specific integrated circuit ("ASIC"), a field programmable gate array ("FPGA") or other programmable logic device, discrete gate or transistor logic, discrete hardware components, or any combination thereof designed to perform the functions described herein. A general purpose processor can be a microprocessor, controller, microcontroller, or state machine, combinations of the same, or the like. A processor can include electrical circuitry configured to process computer-executable instructions. A processor can include an FPGA or other programmable devices that performs logic operations without processing computer-executable instructions. A processor can also be implemented as a combination of computing devices, for example, a combination of a DSP and a microprocessor, a plurality of microprocessors, one or more microprocessors in conjunction with a DSP core, or any other such configuration. Although described herein primarily with respect to digital technology, a processor may also include primarily analog components. For example, some, or all, of the signal processing algorithms described herein may be implemented in analog circuitry or mixed analog and digital circuitry. A computing environment can include any type of computer system, including, but not limited to, a computer system based on a microprocessor, a mainframe computer, a digital signal processor, a portable computing device, a device controller, or a computational engine within an appliance, to name a few.

The elements of any method, process, routine, or algorithm described in connection with the disclosure herein can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of a non-transitory computer-readable storage medium. An exemplary storage medium can be coupled to the processor such that the processor can read information from, and write information to, the storage medium. In the alternative, the storage medium can be integral to the processor. The storage medium can be volatile or nonvolatile. The processor and the storage medium can reside in an ASIC. The ASIC can reside in a user terminal. In the alternative, the processor and the storage medium can reside as discrete components in a user terminal.

Conditional language used herein, such as, among others, "can," "could," "might," "may," "e.g.," and the like, unless specifically stated otherwise, or otherwise understood within the context as used, is generally intended to convey that certain features, elements, and/or steps are optional. Thus, such conditional language is not generally intended to imply that features, elements, and/or steps are in any way required or that one or more embodiments necessarily include logic for deciding, with or without other input or prompting, whether these features, elements, and/or steps are included or are to be always performed. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 10 degrees, 5 degrees, 3 degrees, or 1 degree. As another example, in certain embodiments, the terms "generally perpendicular" and "substantially perpendicular" refer to a value, amount, or characteristic that departs from exactly perpendicular by less than or equal to 10 degrees, 5 degrees, 3 degrees, or 1 degree.

As used herein, "real-time" or "substantial real-time" may refer to events (e.g., receiving, processing, transmitting, displaying etc.) that occur at a same time as each other, during a same time as each other, or overlap in time with each other. "Real-time" may refer to events that occur at distinct or non-overlapping times the difference between which is imperceptible and/or inconsequential to humans such as delays arising from electrical conduction or transmission. A human may perceive real-time events as occurring simultaneously, regardless of whether the real-time events occur at an exact same time. As a non-limiting example, "real-time" may refer to events that occur within a time frame of each other that is on the order of milliseconds, seconds, tens of seconds, or minutes. For example, "real-time" may refer to events that occur within a time frame of less than 1 minute, less than 30 seconds, less than 10 seconds, less than 1 second, less than 0.05 seconds, less than 0.01 seconds, less than 0.005 seconds, less than 0.001 seconds, etc.

Unless otherwise explicitly stated, articles such as "a" or "an" should generally be interpreted to include one or more described items. Accordingly, phrases such as "a device configured to" are intended to include one or more recited devices. Such one or more recited devices can also be collectively configured to carry out the stated recitations. For example, "a processor configured to carry out recitations A, B and C" can include a first processor configured to carry out recitation A working in conjunction with a second processor configured to carry out recitations B and C.

As used herein, "system," "instrument," "apparatus," and "device" generally encompass both the hardware (for example, mechanical and electronic) and, in some implementations, associated software (for example, specialized computer programs for operational control) components.

It should be emphasized that many variations and modifications may be made to the herein-described implementations, the elements of which are to be understood as being among other acceptable examples. All such modifications and variations are intended to be included herein within the scope of this disclosure. Any section headings used herein are merely provided to enhance readability and are not intended to limit the scope of the implementations disclosed in a particular section to the features or elements disclosed in that section. The foregoing description details certain implementations. It will be appreciated, however, that no matter how detailed the foregoing appears in text, the systems and methods can be practiced in many ways. As is also stated herein, it should be noted that the use of particular terminology when describing certain features or aspects of the systems and methods should not be taken to imply that the terminology is being re-defined herein to be restricted to including any specific characteristics of the features or aspects of the systems and methods with which that terminology is associated.

Those of skill in the art would understand that information, messages, and signals may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

While the above detailed description has shown, described, and pointed out novel features, it can be understood that various omissions, substitutions, and changes in the form and details of the devices or algorithms illustrated can be made without departing from the spirit of the disclosure. As can be recognized, certain portions of the description herein can be embodied within a form that does not provide all of the features and benefits set forth herein, as some features can be used or practiced separately from others. The scope of certain embodiments disclosed herein is indicated by the appended claims rather than by the foregoing description. All changes which come within the meaning and range of equivalency of the claims are to be embraced within their scope.

## Claims

1. An electronic device configured to monitor physiological parameters of a user, comprising:
a sensor assembly integrated with the electronic device, the sensor assembly comprising one or more physiological sensors configured to generate physiological data of the user;
a user input unit protruding from a housing of the electronic device, wherein the user input unit is contiguously integrated with an exterior surface of the housing and forms a portion of the exterior surface of the housing, wherein the user input unit is responsive to touch from the user;
a display screen connected to the housing, wherein the user input unit is positioned on the housing out of a plane on which the display screen lies; and
a processor in electrical communication with the display screen, the one or more physiological sensors, and the user input unit,
wherein the processor is configured to cause the display screen to:
display a plurality of display layout previews, wherein the display screen is configured to display less than all of the display layout previews at a time, wherein the display layout previews provide visualizations of display layouts on a reduced scale of size;
move the display layout previews along a path within the display screen responsive to user input via the user input unit, wherein the path comprises an arc of a circle with a center that is offset from a center of the display screen, the center of the display screen being positioned between the center of the circle and the user input unit; and
responsive to user selection of a display layout preview via the electronic device, render a display layout corresponding to the selected display layout preview, wherein the display layout is larger than the selected display layout preview, the display layout comprising indicia of the physiological data originating from the one or more physiological sensors arranged within the display layout according to the selected display layout preview.

2. The electronic device of claim 1, wherein the user input unit does not move relative to the housing.

3. The electronic device of claim 1 or claim 2, wherein the display screen is configured to display no more than two of the display layout previews at a time.

4. The electronic device of any preceding claim, wherein the user input unit is positioned on the housing to the right of the display screen.

5. The electronic device of any preceding claim, wherein the processor is configured to receive the user selection of the display layout preview via the user input unit.

6. The electronic device of any preceding claim, wherein the processor is configured to receive the user selection of the display layout preview via the display screen.

7. The electronic device of claim 5, wherein the user input via the user input unit comprises user contact sliding across the user input unit.

8. The electronic device of any preceding claim, wherein the user input unit comprises a plurality of zones independently responsive to touch from the user, wherein the processor is configured to cause the display screen to move the display layout previews along the path based on user interaction with the plurality of zones.

9. The electronic device of claim 8, wherein the processor is configured to cause the display screen to move the display layout previews along the path with a speed that depends on time between user interactions with the plurality of zones.

10. The electronic device of claim 8, wherein the processor is configured to cause the display screen to move the display layout previews along the path in a direction that depends on an order in which the user interacts with the plurality of zones.

11. The electronic device of any preceding claim, wherein the user input unit has a length between about 25% and about 75% a diameter of the display screen.

12. The electronic device of any preceding claim, wherein the user input unit has a height between about 0.4cm and 0.6cm.

13. The electronic device of any preceding claim, wherein the user input unit is one of a capacitive, resistive, infrared, surface capacitive, or projected capacitive touch (PCAP) user input unit.
